Europäisches Patentamt

European Patent Office

Office européen des brevets

(19)

(11) Publication number: **0 018 218**
**B1**

(12)

# EUROPEAN PATENT SPECIFICATION

(45) Date of publication of patent specification: **23.11.88**

(21) Application number: **80301258.2**

(22) Date of filing: **18.04.80**

(51) Int. Cl.⁴: **A 61 K 45/02** // A61K39/12, C12N1/00, C12N15/00

(54) Interferon proteins and method of producing same.

(30) Priority: 20.04.79 DK 1645/79
22.02.80 DK 791/80
02.04.80 DK 1484/80

(43) Date of publication of application:
29.10.80 Bulletin 80/22

(45) Publication of the grant of the patent:
23.11.88 Bulletin 88/47

(84) Designated Contracting States:
AT BE CH DE FR GB IT LI LU NL SE

(56) References cited:
EP-A-0 032 134
EP-A-0 060 057
DE-A-2 722 970
DE-A-2 812 989
GB-A-2 037 296

Chemical Abstracts, vol. 89, no. 5, 31 July 1978, Columbus, Ohio, USA, K.P. FUNG et al. "Heterocomplexes of human interferon and immunoglobulins: formation and properties", p. 409, abstract no. 40691v

Chemical Abstracts, vol. 90, no. 25, 18 June 1979, Columbus, Ohio, USA, G. BODO "Production and pruification of human lymphoblastoid interferon", p. 454, abstract no. 202028j

(73) Proprietor: Technobiotic Ltd.
Töpferstrasse 5
CH-6004 Lucerne (CH)

(72) Inventor: Berg, Kurt Frimann
Paul-Petersensvej 5
DK-2820 Gentofte (DK)

(74) Representative: Walls, Alan James
c/o Simmons & Simmons 14 Dominion Street
London EC2M 2RJ (GB)

(56) References cited:
Chemical Abstracts, vol. 90, no. 21, 21 May 1979, Columbus, Ohio, USA, M. RUBINSTEIN et al. "Human leukocyte interferon: Production, purification to homogeneity. and initial characterization", p. 420, abstract no. 166337y

Chemical Abstracts, vol. 86, no. 11, 14 March 1977, Columbus, Ohio, USA, S. PAHLMAN et al. "Hydrophobic interaction chromatography on uncharged sepharose derivatives. Effects of neutral salts on the adsorption of proteins", p. 232, abstract no. 67745r

Courier Press, Leamington Spa, England.

(56) References cited:

G. Bodo, "Production and Purification of Human Lymphoblastoid Interferon", Int. Immunobiol. Symp., (Proc.), vol. 11, pp. 49-57, 1977

Proc. Natl. Acad. Sci., USA, vol. 76, no. 2, pp. 640-644, February 1979

Analytical Biochemistry 65, pp. 369-379 (1975)

Analytical Biochemistry 99, pp. 170-174, (1979)

Analytical Biochemistry 67, pp. 55-65, (1975)

J. Biol. Chem. 246, no. 14 4504-4509 (1971)
Biochem. and Biophys. Res. Comm. 45 no. 3 662-668 (1971)

Anal. Biochem. 9 230-235 (1978)

Int. J. Oral. Surg. 14 184-194, (1985)

Anal. Biochem. 109 76-86 (1980)

Anal. Biochem. 93 153-157 (1979)

Anal. Chem. 28 870-873

Anal. Biochem. 67 503-506

**Description**

The present invention relates to the purification of human Le form interferon, to a purified form of human Le form interferon, and to purification and preparative methods relevant thereto.

As used herein, the term "protein" includes "glycoprotein".

Many attempts have been made to purify human interferon. The objectives of such purification attempts have included a complete characterization of the interferon species for standardization purposes. To date, none of the attempts to purify human Le form interferon have been completely successful.

This invention is based on the discovery of purification methods which permit the preparation, for the first time, of all the components of human Le form interferon protein substantially free of inactive and otherwise undesirable impurities.

The Le form of interferon is defined in a paper by E. A. Havell, B. Berman, C. A. Ogburn, K. Berg. K. Paucker, and J. Vilcek, Proc. Nat. Acad. Sci. USA, 72, 2185—2187 (1975).

According to the invention, pure human leukocyte interferon proteins have been prepared from crude human leukocyte interferon through a number of special purification steps, and the pure human leukocyte interferon has been characterized by stained protein bands in SDS PAGE (sodium dodecylsulfate polyacrylamide gradient electrophoresis).

The particular experimental conditions used for the first preparation and characterization of the pure human leukocyte interferon proteins appear from the below sections "Materials and Methods" and "Experimental Section". Some of the products and procedures involved in the preparation and characterization of the pure interferon proteins are novel *per se* and constitute aspects of the invention of general applicability within interferon technology and, in a broader sense, in protein purification technology. The pure human interferon proteins, and especially, pure human Le form interferon proteins, now made available and characterized according to the invention, are in themselves aspects of the invention and constitute the key to further new developments which are also aspects of the invention and which are explained and illustrated in the present specification.

In several repeated experiments, it has been established that under the SDS PAGE and staining conditions described in the section "Materials and Methods", at a total interferon load of $0.9 \times 10^6$ IFU, pure human leukocyte interferon shows essentially only two sharp stained protein bands at $18,400 \pm 200$ and $20,100 \pm 200$ Daltons, respectively, and a minor stained protein band between $20,300 \pm 200$ and $20,400 \pm 200$ Daltons. As determined by the protein determination described below, the pure human leukocyte interferon has a specific activity of about $10^9$ IFU per mg of protein; the specific activity found may vary to some extent depending upon the protein determination method employed, and the specific activity on a protein weight basis is judged to be $2 \times 10^8$—$2 \times 10^9$ IFU per mg of protein. The fact that the pure interferon shows two major distinct bands is in accordance with prior art findings using crude or partially purified interferon preparations which indicated that human leukocyte interferon comprises at least two major species. At a higher total interferon load, e.g., of $3.8 \times 10^6$ IFU, the above-mentioned SDS PAGE system has been found to be capable of showing a more differentiated protein pattern comprising six interferon protein bands, i.e. the two strongly stained bands at $18,410 \pm 200$ Daltons and $20,180 \pm 200$ Daltons, respectively, a medium-stained band at $20,420 \pm 200$ Daltons (corresponding to the above-mentioned minor stained band) and just visible protein bands at $19,500 \pm 200$ Daltons, $21,130 \pm 200$ Daltons, and $23,440 \pm 200$ Daltons, respectively. Each of the individual components in the above-mentioned bands of the SDS PAGE acrylamide gradient gel has been found to show biological interferon activities: antiviral activity, ability to neutralize only anti-human leukocyte interferon (but *not* anti-human fibroblast interferon), and anticellular activity, plus a variety of so-called non-viral activities, as exemplified by potentiation of Natural Killer cells, potentiation of MLC-CML, increase of HLA antigens, etc. The complete purification of interferon proteins makes it possible, for the first time, to produce anti-interferon which is strictly specific to the active species simply by immunizing animals with the pure interferon preparation or one or more of its components. Such strictly monospecific anti-interferon is extremely useful for antibody affinity chhromatography for purification of crude or partially purified interferon to obtain, in a simple and economic way, large amounts of pure interferon or highly purified interferon for clinical purposes, standardization, chemical studies, sequence studies, and as immunogen for repeated preparation of monospecific anti-interferon. It is within the scope of the present invention not only to purify human leukocyte interferon by means of the monospecific antibody raised against the pure human leukocyte interferon, but also to purify other interferon types which cross-react immunologically with the monospecific anti-interferon, e.g. "Namalva" interferon (human lymphoblastoid interferons; the Le form interferon constitutes about 85% of the biological activity of human lymphoblastoid or Namalva interferon, vide E. A. Havell, Y. K. Yip, and J. Vilcek, "Characterization of human lymphoblastoid (Namalva) interferon", J. gen. Virol., 38, 51—59 (1977)), and interferon containing the Le form obtained by cultivation of a microorganism carrying DNA coding for the production of interferon proteins (or proteins having the significant biological interferon activity determinants).

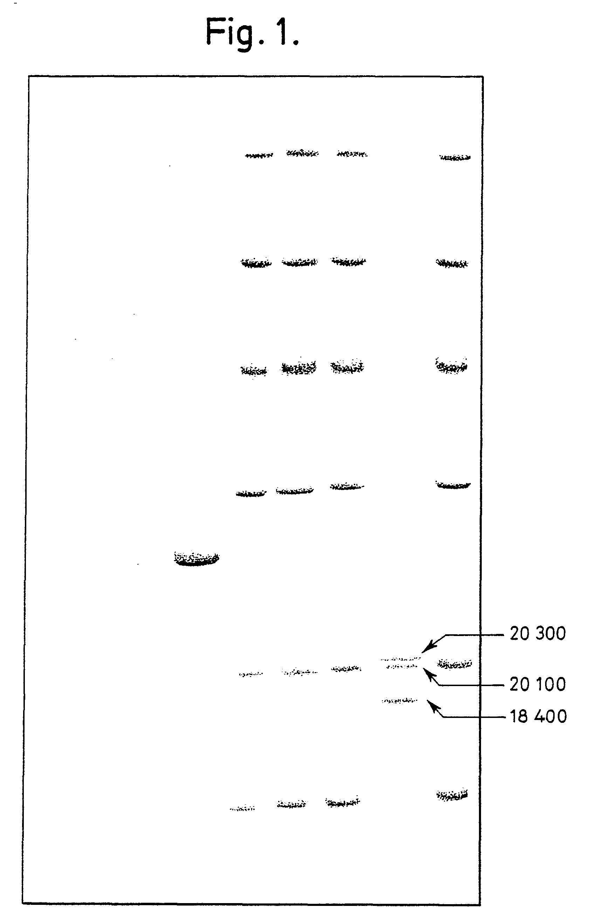

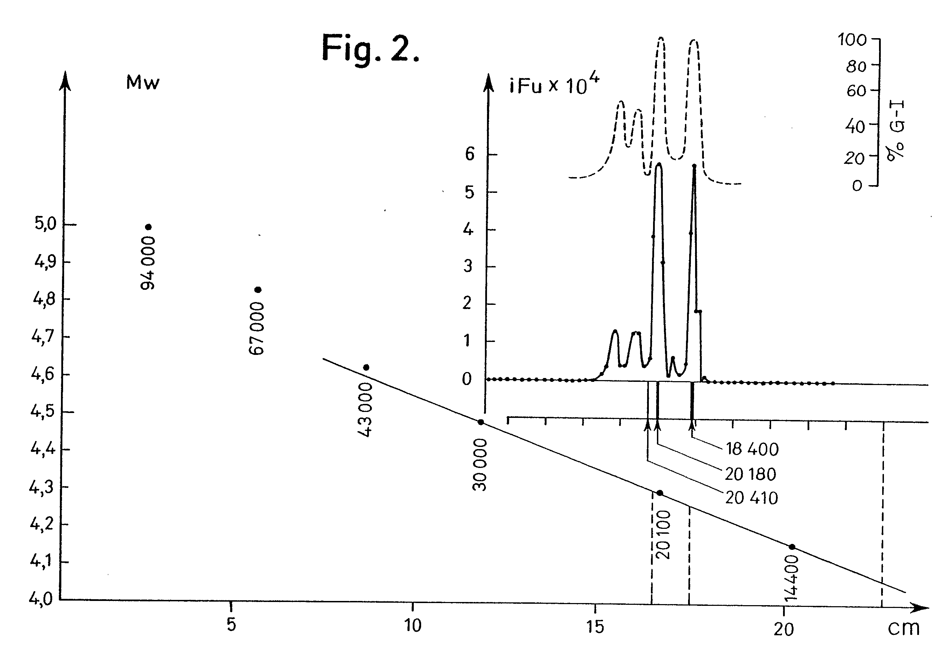

At the interferon load of $0.9 \times 10^6$ IFU, the pure human leukocyte interferon proteins appear as the above-mentioned three individual protein bands in the SDS PAGE acrylamide gradient gel, together with five—six biological peaks. Whether five or six biological bands are found depends on the exact places at which the gel slice is cut. Reference is made to Fig. 1 which shows a stained SDS PAGE gradient gel slab prepared at this load as described in the section "Materials and Methods" below. Each of the protein bands has been shown to possess distinct interferon activity. Reference is made to Fig. 2 which is a drawing of an SDS slab from another experiment at the same interferon load, Fig. 2 also showing the interferon activity profile associated with the bands, determined as explained under "Materials and Methods" below. Five biological interferon peaks are seen, together with three distinct stained proteins.

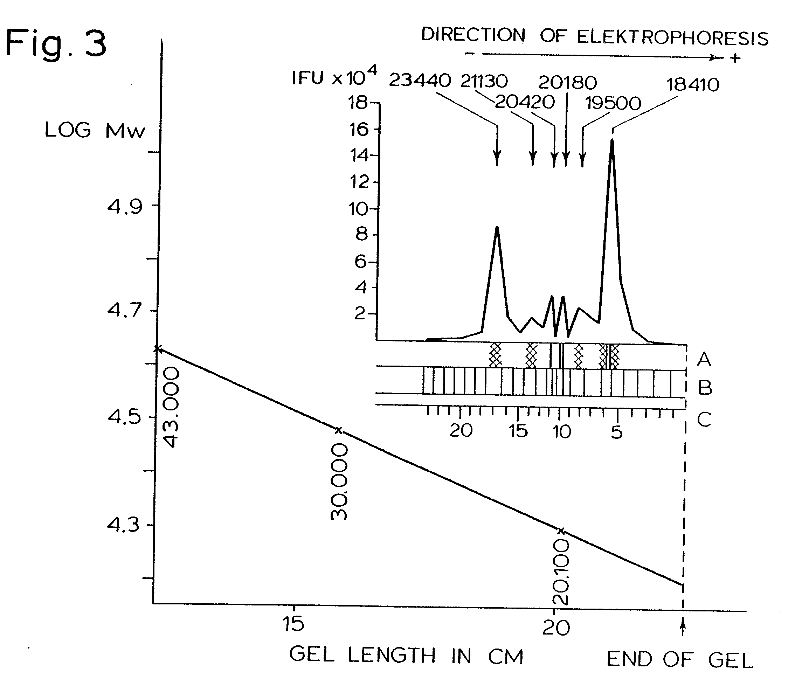

From Fig. 2 is can be seen, umambiguously, that the protein bands coincide strictly with the peaks of interferon activity. This proves that the proteins are interferon proteins. It is important to note that the interferon activity profile will, of course, depend upon the exact position of the invididual slicings of the gel. In Fig. 2, the individual interferon activity of the minor band at 20,410±200 Daltons is not so evident, but in other experiments at the same interferon load, it was shown that the minor band itself posssesses interferon activity, and in experiments with a higher load, vide below, the minor band was found to be a distinct interferon sub-species. The amount of interferon activity from the SDS PAGE found in the corresponding interferon protein slices corresponds linearly with the amount of protein as assessed from the intensity of the staining of the protein bands. Thus, the unambiguous existence of the two major interferon proteins and the minor band has been demonstrated in the experiments illustrated in Figs. 1 and 2. In experiments where the interferon load in the system was higher, the above-mentioned more detailed band pattern was demonstrated, such as illustrated in Fig. 3 (six interferon proteins together with six biological peaks determined after staining and destaining). As it is known that interferon treated with SDS will retain its immunological determinants and even expresses (or preserves) its antigenicity in a more distinct way compared to non-SDS-treated interferon (as shown by immunizations of mice with human leukocyte interferon preparations of Paucker et al. (Dalton, B.f., Ogburn, C.A., Paucker, K., Production of antibodies to human interferons in mice, Infect. Immun. *19*(2), 570—574 (1978), pp. 4; 25—30), preparative SDS PAGE makes it possible to not only obtain each of the components in isolated form, but also to perform immunization with the isolated components, such as illustrated in greater detail below.

Expressed with reference to specific activity, the invention relates to human interferon or species thereof having a specific activity of about $2 \times 10^8$—$2 \times 10^9$ IFU per mg protein. However, since the methodology concerning the protein determination varies considerably, the actual figure of the specific activity is of less importance compared to the clear demonstration, by SDS PAGE, of the individual species.

The pure interferon proteins of the invention are, therefore, more suitably expressed as human Le form interferon proteins which under the SDS PAGE and staining conditions defined herein at a total interferon load of $0.9 \times 10^6$ IFU show two major sharp stained protein bands having antiviral interferon activity at 18,400 and 20,100 Daltons, respectively, and a minor stained protein band having antiviral interferon activity between 20,300 and 20,400 Daltons, together with smaller peaks of antiviral interferon activity at 19,500, 21,130, and 23,440 Daltons (said Dalton molecular weights being subject to an experimental accuracy of ±200 Daltons), said SDS PAGE acrylamide gradient showing essentially no other stained protein regions; or as human Le form interferon proteins which under the SDS PAGE and staining conditions defined herein at a total interferon load of $3.8 \times 10^6$ IFU show six stained protein bands having antiviral interferon activity, viz. strong bands at 18,410 Daltons and 20,180 Daltons, respectively, a medium-strong band at 20,420 Daltons and just visible bands at 19,500 Daltons, 21,130 Daltons, and 23,440 Daltons, respectively (said Dalton molecular weights being subject to an experimental accuracy of ±200 Daltons), the peaks of antiviral interferon activity coinciding exactly with the stained protein bands, said SDS PAGE acrylamide gradient showing essentially no other stained protein regions.

It is important to note that the individual components in the above-mentioned bands of the SDS PAGE gel show biological interferon activity, ability to neutralize anti-human leukocyte interferon, and anticellular activity, etc. The invention also relates to each of the individual components represented by each of the above-mentioned individual SDS PAGE bands, as well to any protein having the significant biological interferon activity determinant(s) possessed by the individual components, and to any protein having the significant immunological determinant(s) possessed by the individual components.

With respect to origin, the human interferon proteins of the invention may be derived from human leukocyte interferon prepared using human cells or from cultured human lymphoblastoid (Namalva) cells, or from proteins prepared by cultivation of a microorganism containing DNA coding for the interferon or an important part thereof, such as described above, but also human Le form interferons of other origin, but conforming with the above characteristics, are within the scope of the present invention.

It is well known that human Le form interferon shows a number of important therapeutic aspects in man, including antiviral and anti-tumor activity, and the provision of the pure human Le form interferon makes it possible to further exploit these useful properties. One aspect of the invention comprises a formulation comprising the pure human Le form interferon protein or proteins adapted for administration to human beings or animals for prophylactic, therapeutic, or immunization effect. Such a formulation may, e.g., be adapted for parenteral, intranasal, or topical administration.

A most useful formulation of the pure interferon proteins of the present invention is an aqueous solution. Pure interferon proteins in aqueous solution should be stabilized, and the choice of stabilizer will depend upon the use of the solution. When the solution is to be used for administration to human beings, e.g., parenteral administration, the stabilizer should be a physiologically acceptable stabilizer, and a suitable stabilizer is a protein or combination of proteins which is non-toxic and non-immunogenic in human beings, such as human serum proteins and fractions thereof, and human albumin. A typical preferred stabilizer is 1% human albumin. The normal concentration of pure interferon proteins in compositions for parenteral administration to human beings will be in the range corresponding to 1—20 million IFU per ml, and a normal daily dose will be 3 to 10 million, e.g. 5 to 10, million IFU totally, preferably administered once or twice a day by intramuscular injection. When preparing solutions of pure interferon for administration to human beings, normal pharmaceutical precautions which are customarily taken in connection with the preparation of parenteral compositions, will also be observed, such as precautions to ensure sterility and freedom from pyrogenicity.

When the stabilized formulation of the invention is an aqueous solution of pure human Le form interferon protein(s) to be used for immunization of animals for the preparation of monospecific anti-interferon claimed in European Patent Application No. 83101471.7, stabilization with SDS (sodium dodecylsulfate) to form an SDS complex of the human Le form interferon protein(s) is a preferred stabilization in view of the above-mentioned fact that SDS increases the antigenicity and/or stability of interferon. As explained in greater detail below, the pure interferon-SDS combination or complex may be formed simply by adding SDS to the aqueous pure interferon proteins, preferably in a concentration of about 0.1% by weight, calculated on the solution, at pH 7.2. The SDS complex of the human Le form interferon protein or proteins constitutes, in itself, a valuable aspect of the present invention because of the stability thereof, and a most interesting form of such complex, well suited for storage and transport (suitably at low temperature, e.g., at a temperature of at the most 4°C or preferably −20°C, is when isolated in solid form such as described below.) The use of other stabilizers of the detergent type for this purpose is within the scope of the present invention. A further preferred form of the pure human Le form interferon proteins is a form in which they are bound to Cibacron Blue F3GA or another ligand capable of binding the interferon proteins according to the mechanism exhibited by Cibacron Blue F3GA, such as will be explained in greater detail below.

The pH of the pure interferon protein solution for immunization of animals to prepare the monospecific anti-interferon is preferably about 7.2, and a suitable buffer is PBS (phosphate buffered saline).

The stabilized pure interferon protein preparation for immunization of animals may additionally comprise an adjuvant to further increase the antigenicity, and one suitable adjuvant is Freund's adjuvant.

It is also within the scope of the invention to increase and/or stabilize the antigenicity of the pure Le form interferon proteins or each member thereof by coupling to an immunogenic carrier (so as to present the pure interferon protein or proteins as a sort of "hapten") in accordance with wellknown principles. As examples of immunogenic carriers may be mentioned PPD (Purified Protein Derivative) and BCG (Bacille Calmette Guérin). However, the use of such immunogenic carriers is not presently preferred.

As the stained interferon protein bands in the SDS PAGE have preserved their antigenicity completely or to a considerable extent, it is also possible to use the stained interferon proteins directly cut out from an SDS PAGE as antigen preparations for immunizing immunizable animals such as rabbits.

Interestingly, it has been found that antibodies raised against one of the purified interferon proteins of the invention are capable of neutralizing the other purified proteins of the invention. Thus, as will become apparent, the monospecific antibodies (which are claimed in European Patent Application No. 83101471.7), whether raised against a single purified interferon protein of the invention or raised against a combination of purified interferon proteins of the invention, are equally effective for purification of human Le form interferon-containing solutions.

In accordance with wellknown principles, the monospecific anti-interferon can be used for determination of the corresponding interferon or interferon component in biological fluids such as by radioimmunoassay or related techniques. The monospecific antibodies may be employed antibody affinity chromatography purification of interferon-containing solutions to prepare the purified Le form interferon proteins of this invention. For this purpose, the antibodies are immobilized on a matrix in a manner known *per se*, suitably covalently bound to a suitable antibody affinity chromatography matrix such as a cross-linked agarose such as Sepharose 4B from Pharmacia. The antibody affinity chromatography purification of interferon-containing solutions may be performed according to any of the wellknown methods, either batchwise or, preferably, using the matrix-immobilized antibody arranged in a column.

The preparation of antibody affinity columns using the monospecific anti-interferon, and the operation of such columns are performed in a manner known *per se*. The interferon-containing solution applied on such columns may be a crude, unconcentrated interferon preparation, or it may be a concentrated or partially purified interferon preparation. The interferon preparation applied on the column may be any interferon preparation containing human Le form interferon, that is, human leukocyte interferons, human lymphoblastoid interferons (Namalva interferons), or interferon (or important parts thereof) produced by cultivation of a microorganism containing DNA coding for interferon, such as described above. The use of antibodies against partially purified human leukocyte interferon in antibody affinity chromatography for purifying Namalva interferon and leukocyte interferon has already been described (vide, e.g. Scand. J. Immunol., *8* 429—436 (1978)). However, the important improvement is that monospecific anti-interferon will retain substantially only human Le form interferon protein, the remaining proteins of the preparation passing through the column. Very small amounts of impurities due to spontaneous cross-reactivity cannot be ruled out, not even when the antibodies used are antibodies produced by hybridoma technique which must, apart from this, be expected to "produce" (react with) only pure interferon proteins.

At suitable dimensions of such antibody columns (which can be designed in accordance with wellknown principles for antibody affinity chromatography columns), the columns may be used for large scale industrial purification of interferon from a crude interferon preparation to result in pure (or highly purified) interferon proteins in the column eluate. The pure (or highly purified) interferon proteins prepared in this way are stabilized with suitable stabilizers according to the intended use thereof, such as described above.

As the interferon of the interferon preparations applied on the monospecific anti-interferon columns is present in usually very low concentrations, on a weight basis, and as as great amounts as possible of the valuable interferon are to be isolated, it is of importance to minimize any deterioration of the interferon proteins which might be caused due to the presence of proteolytic activity in any biological substance with which the interferon is contacted.

Thus the removal of proteolytic activity from the anti-interferon antibodies (immunoglobulins) is suitably performed by treating the antibodies, prior to their binding to the matrix, with matrix-immobilized enzyme inhibitor or enzyme destructor which is not harmful to immunoglobulins (or the important fragments thereof). Thus, the antibodies may be passed through a column of matrix-immobilized poly-L-lysin and/or matrix-immobilized Soyabean Trypsin inhibitor, and/or matrix-immobilized kallikrein inactivator. An example of a suitable treatment of the antibodies is passage through a column of poly-L-lysin covalently bound to cross-linked agarose such as Sepharose 4B, followed by passages through a column of Soyabean Trypsin inhibitor covalently bound to the same matrix. It has been found that this removal of proteolytic activity increases the recovery of interferon activity in antibody affinity chromatography purification of interferon-containing solutions.

The monospecific anti-interferon, when covalently bound to a matrix such as cross-linked agarose, is preferably bound to such an extent that the total amount of antibody covalently bound to the matrix corresponds to at the most 85% of the immunoglobulins used at the covalent binding stage, such as described by the present inventor in Scand. J. Immunolog., 6, 77—86 (1977). This results in the highest recovery of interferon from the column.

When the eluate from the monospecific anti-interferon affinity chromatography column is to be used for administration in human beings, it is important that it does not contain any component which might be immunogenic in man. One risk which might be associated with antibody affinity chromatography is that immunoglobulins or immunoglobulin fragments liberate from the column and become eluted together with the desired protein or proteins.

Such immunoglobulins or fragments thereof which are immunogenic in man are removed by passage of the eluate through an antibody affinity column in which the antibodies are directed against the anti-interferon immunoglobulins and are of a kind which is non-immunogenic on parenteral administration to human beings. (Prior to the passage of the eluate through the said column, it should be adjusted to a neutral pH, e.g. by dialysis against PBS, pH 7.2).

The purification stages performed according to the present invention to prepare the pure human leukocyte interferon proteins (human Le form interferon proteins) from crude human leukocyte interferon comprise concentration by precipitation of proteins with KSCN, gel filtration, ligand affinity chromatography, and antibody affinity chromatography. Although such stages are known *per se* in the interferon art, the particular combination thereof and the particular conditions applied in certain of the operations constitute novel features, some of which are in themselves aspects of the present invention. The particular way in which the stages are performed and the particular combination of operations have resulted in optimal purification and concentration of the interferon, with minimum loss of interferon proteins during the sequence.

The KSCN precipitation is preferably performed by lowering the pH of the crude interferon containing a KSCN concentration of 0.5 M to pH 4.5 instead of the conventional lowering to pH 3.5. This results in a considerably lower amount of protein in the precipitate, thus facilitating the later purification steps.

The gel filtration is performed with a buffer solution containing 25% by volume of ethylene glycol and being 1 molar with respect to NaCl, incl. PBS (pH 7.2). This results in a much better resolution than when using PBS or low pH (2,4) alone, or when using urea, PBS at pH 7.2. The eluate fractions containing essentially only proteins in the 10,000—20,000 Daltons range are collected.

The ligand affinity chromatography is performed in a novel and extremely advantageous way and constitutes one important aspect of the present invention:

The said ligand affinity chromatography is performed under specified conditions on an interferon having a specific activity of at least 50,000—100,000 IFU per mg protein, using immobilized Cibacron Blue F3GA as the ligand. The use of Cibacron Blue F3GA as the ligand for affinity chromatography of interferon was known in the art, but according to the invention, it has been found that the selectivity of this ligand increases drastically when particular combinations of conditions are used: the interferon applied should have a much higher specific activity i.e. a specific activity of at least 50,000—100,000 IFU per mg protein, than in the conventional uses of this ligand type (where crude human leukocyte interferon of a specific activity of about 3—5×10³ IFU per mg of protein is applied), and the solution in which the interferon is applied on the column should be in the pH range of 6.5—8 and should have a ionic strength which does not essentially exceed the ionic strength of a 10—100, in particular 20, mM phosphate buffer, pH 7.2. When such a relatively high specific activity of the interferon is applied, the specificity of the ligand changes, and a higher degree of selective binding of the interferon proteins to the ligand occurs. Cibacron F3GA is believed to interact with interferon proteins in a way which indicates the existence of a "dinucleotide fold" and in this interaction, it seems to have the same binding site as polyribonucleotides. It is believed that the particular advantageous properties shown by Cibacron F3GA under particular critical conditions as discussed above will also be exhibited by the other members of the class to which this ligand pertains, and the present aspect of the invention, therefore, is constituted by a method of purifying humand interferon, comprising applying an aqueous solution containing human Le form interferon protein in a form having a specific activity of at least 50,000—100,000 IFU per mg of protein, the said solution being buffered to a pH of 6.5—8 and having an ionic strength substantially not exceeding the ionic strength of a 10—100, in particular 20, mM phosphate buffer, pH 7.2 solution, optionally together with a water miscible organic solvent such as ethanol in amounts of 5—80%, on a matrix-immobilized ligand capable of binding the interferon according to the mechanism exerted by Cibacron F3GA, and thereafter eluting the interferon thus bound.

Examples of materials which are matrix-immobilized Cibacron F3GA are "Blue Dextran 2000" (matrix: dextran with molecular weight 2 millions), and Blue Sepharose CL-6B. Further details concerning these and other materials and their use in the conventional interferon purification appear from Bolin et al., Preparative Biochemistry, 8(4), 259—274 (1978).

According to the invention, it is preferred to use, as the immobilized Cibacron F3GA composition, Blue Dextran 2000 coupled to Sepharose 4B (by means of CNBr-activated Sepharose 4B).

The elution of the interferon from this type of immobilized ligand has been found, according to the invention, to be extremely selective when using 0.6 M NaCl buffered to pH 7.2, and pH 7.2 is also the preferred pH of the interferon-containing solution applied.

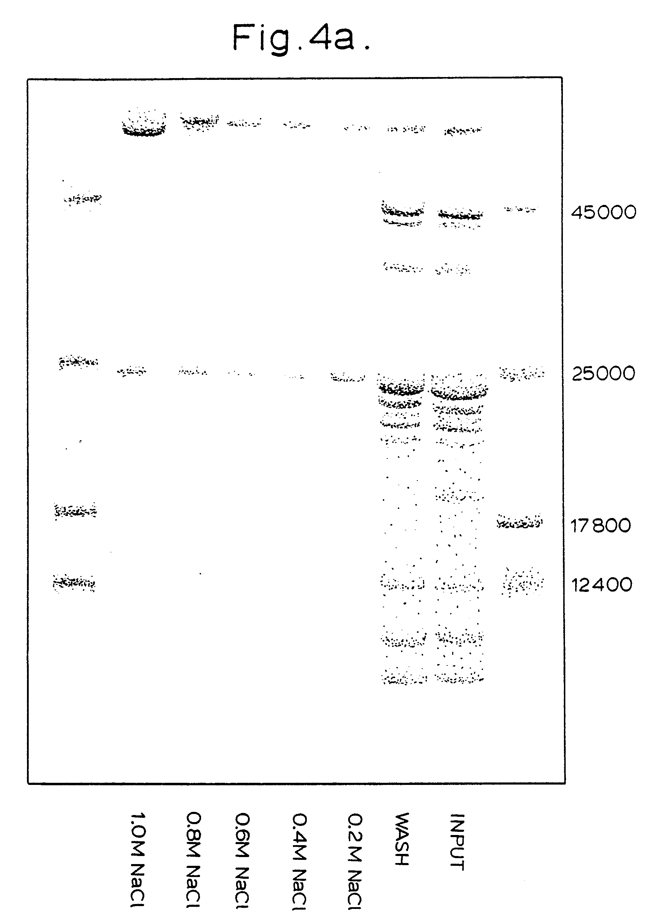

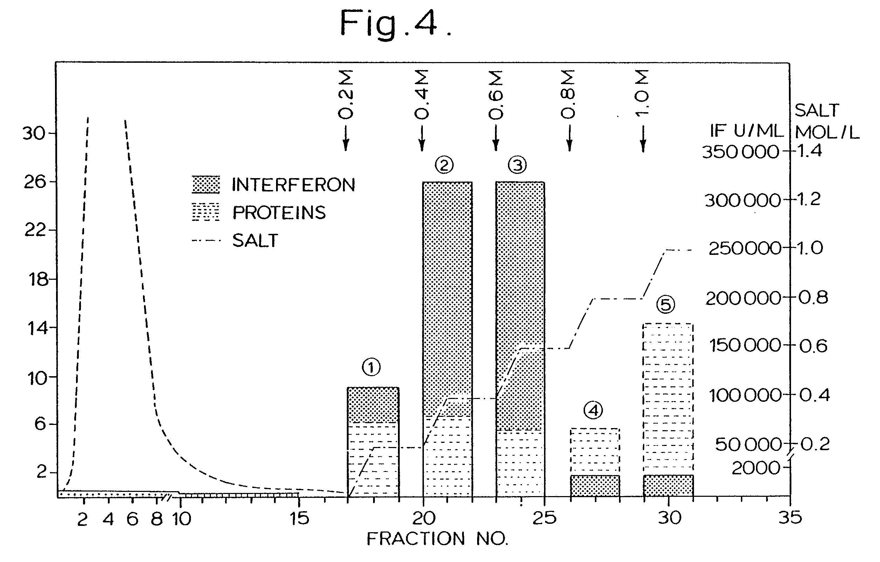

Reference is made to Fig. 4 which shows the elusion pattern of a Blue Dextran-Sepharose 4B column loaded with partially purified human leukocyte interferon, 1 ml, specific activity 500,000 IFU per mg protein, subsequent to throughout dialysis versus 20 mM phosphate buffer (PB), pH 7.4. The size of the fractions was 5 ml, and the flow rate was 35—40 ml/h. The column was washed with 20 mM PB for 2 hours, before it was eluted stepwise with 0.2, 0.4, 0.6, 0.8, and 1.0 M NaCl in PB 7.4, respectively. The total eluate (I+II+III) contained 754,000 IFU (in 30 ml), the originally applied amount being determined to 750,000 IFU. Hence, the recovery was 100%. The specific activity of the eluate was $2.1 \times 10^7$ IFU per mg of protein. The purification factor was 42. When checking the eluates in an SDS PAGE, most of the eluted proteins (>98%) appeared above 50,000 Daltons (impurities), vide Fig. 4a which shows an SDS PAGE of the input, wash, and eluate of Fig. 4. Although, as will appear from the above, 0.6 M NaCl buffered to pH 7.2 is a most preferred eluant for the affinity column, it will also be noted that a broader concentration range is quite selective, and the invention comprises the elution with aqueous NaCl solution of a concentration of 0.5—0.7, in particular 0.5—0.65 molar and buffered to a pH of 6.5—8, or other aqueous solution buffered to a pH of 6.5—8 and having an ionic strength corresponding to such NaCl solution. The use of other eluants is also within the scope of the present invention. As examples may be mentioned salts and/or ethylene glycol in stepwise and/or gradient-wise increasing concentration up to 50%, aminoacids, artificial aminoacids, ampholines, and proteins and protein mixtures. As mentioned above, the interferon solution may be applied together with a water miscible organic solvent, such as alcohol, in particular ethanol.

The interferon which is purified by the affinity chromatography according to this aspect of the invention is typically an interferon containing human Le form interferon proteins, such as human interferons (apart from human fibroblast interferons), that is, e.g., humand leukocyte interferons, human lymphoblastoid interferons and human Le form interferon proteins or important parts thereof when produced by cultivation of a microorganism clone containing DNA coding for the production of such interferon protein. (The fact that human lymphoblastoid interferon (Namalva) contains a minor proportion of interferon of fibroblast character (F form—corresponding to 15% of the biological activity) does not detract from the fact that human lymphoblastoid interferon is, with respect to its major interferon activity, a human Le form interferon in that it contains human Le form interferon proteins (corresponding to 85% of the biological activity) having determinants identical with determinants of human leukocyte interferon proteins, such as has been shown according to the present invention.)

It is preferred that the specific activity of the interferon preparation applied on the affinity column is 100,000—1,000,000, such as 200,000—1,000,000, e.g. about 500,000, e.g. 500,000—1,000,000 IFU per mg protein.

The eluate from the affinity chromatography column operated in accordance with this aspect of the invention may be an interesting product also for therapeutic use. It will often have a specific activity of at least $30 \times 10^6$ IFU per mg protein, based on the Lowry procedure using pure human albumin serum as standard, such as $30 \times 10^6$—$10^8$, e.g. $30 \times 10^6$—$70 \times 10^6$ IFU per mg protein. For administration to human beings, this preparation is subject to normal pharmaceutical precautions, such as precautions to ensure sterility and freedom from pyrogenicity. The dosage of this preparation will correspond to the dosage stated above for the pure interferon, on a total activity basis.

As explained in the "Experimental section", the eluate from the affinity chromatography column was, in the original experiments leading to the pure interferon, subjected to final purification by passage through an absorbed antibody affinity column in which the antibodies are immunoglobulins raised against partially purified human leukocyte interferon and then subjected to removal of antibodies against contaminating proteins by several passages through columns of matrix-immobilized crude human leukocyte interferon. As appears from the more detailed explanation below, the covalent binding of crude interferon to a matrix (as e.g. Sepharose 4B) destroys the immunological determinants of the interferon itself, (>98%), but apparently not the determinants of the major part of the impurities, and this means that when immunoglobulins raised against partially purified leukocyte interferon are passed (normally several times) through the column, the anti-impurities thereof will be retained on the column, while the anti-interferon will pass the column. Such absorbed anti-interferon (absorbed several times) was used in the antibody affinity chromatography stage following the affinity chromatography.

The antibodies used in the antibody affinity column may themselves be obtained by immunizing an immunizable animal with the individual protein components of the invention, e.g. as cut from the SDS PAGE gel.

As appears from the "Experimental section", a preferred way of operating the affinity columns, that is, the Blue Dextran Sepharose column and the antibody affinity column, is to connect the two columns so that the eluate from the Blue Dextran column at the same time loads the antibody affinity column. This prevents any loss which might otherwise occur if the eluate fractions from the Blue Dextran column were handled separately.

In the final concentration of the human Le interferon proteins, a unique method of concentrating proteins by precipitation with SDS was used. This method constitutes a further aspect of the present invention and comprises precipitating SDS or a salt thereof from a solution of the protein which contains SDS, preferably in a concentration of 0.1—4 per cent by weight, in particular about 0.1 per cent by weight, to obtain a precipitate comprising a complex or complexes of SDS or a salt thereof with the protein, separating the precipitate from the solution, preferably by centrifugation at 0—4°C, and redissolving the precipitate in a smaller liquid volume. The precipitation of the SDS may suitably be obtained by either a) lowering the temperature to 0° for about 15 minutes or b) adding a salt, e.g., a $K^+$ salt, which forms a precipitate with SDS or with SDS-protein complexes. This method is a valuable method for concentrating aqueous solutions of pure or purified interferons, and, as indicated above, has been found to be an excellent way of concentrating human Le form interferon proteins.

The total purification sequence performed in accordance with the present invention was found to be extremely activity-preserving: From a starting amount of proteins of $7 \times 10^5$ gamma, the pure interferon isolated was less than or equal to 1 gamma, (as determined by comparison of protein bands on SDS PAGE). Yet, the overall decrease in total interferon activity from the starting batch of crude interferon to the pure interferon was only from $4 \times 10^6$ IFU to $1.85 \times 10^6$ IFU (about 50%). This emphasizes the unique character of the purification sequence and the above-mentioned critical stages thereof.

Materials and methods

Interferon assays were performed according to the well-known standard method (Berg K., Sequential Antibody Affinity Chromatography of Human Leukocyte Interferon, Scand. J. Immunol. 6, 77—86 (1977)) using VERO cells (monkey kidney cells) and Vesicular Stomatitis Virus (VSV) as a challenge virus. All interferon units (IFU) are expressed in international reference units (69/19 B units) (69/19 B reference was obtained from MRC, Mill Hill, U.K.).

Interferon. Crude human leukocyte interferon was produced according to the method as described by Cantell (Cantell, K., Hirvonen, S., Mogensen, K. E. and Pyhälä, L., Human leukocyte interferon: production, purification, stability and animal experiments. In: The Production and use of Interferon for the Treatment and Prevention of Human Virus Infections pp. 35—38, Waymouth, C. (ed.). Proceedings of a Tissue Culture Association Workshop held at Lake Placid, 1973 (In Vitro Monograph, volume 3), Tissue Culture Association, Rockville, Md.) using Sendai virus as interferon inducer. Partially purified interferon (PIF) with a specific activity of $5 \times 10^5$ IFU/mg protein was obtained from crude concentrated human leukocyte interferon (CIF) by ethanolic precipitation as described by Cantell, K., Hirvonen, S., Mogensen, K. E. and Pyhälä, L., loc. cit.

Crude Namalva interferon was produced substantially as described by Strander et al., Production of human lymphoblastoid interferon, J. Clin. Microbiol. 1, 116—124 (1975), using Sendai virus as interferon inducer.

Interferon neutralization for determining anti-interferon was performed in a micro-assay system in the following manner: 20,000 VERO cells per well were seeded in 100 µl medium and kept at 5% $CO_2$ in a humidified cabinet. On day 2 the medium was removed from the cells, and each well received 100 µl of a dilution (in medium) of the antiserum, containing an interferon concentration of 6—8 IFU/ml (the serum and interferon had been preincubated at 37°C for 1 h). On day 3 the medium was removed, and all the wells received 100 µl VSV (diluted to $10^{-3.5}$ in medium). On day 4 the CPE (cytopathogenic effect) was determined, and 50% destruction was taken as the end point for the determination of the anti-interferon titer. The titers are expressed as interferon neutralization units (IFU-NU) per ml.

Non-monospecific anti-interferon against PIF was produced, according to Mogensen, K. E., Pyhälä, Liisa and Cantell, K., Acta path. microbiol. Scand. Sect. B, 83, 443—450, (1975), partly is sheep, partly in rabbits. The titer of the sheep anti-interferon was 100—250,000 IFU-NU/ml. For the preparation of the rabbit anti-interferon, a rabbit was injected weekly, s.c. with PIF ($2 \times 10^5$ IFU) for more than two years. The titer of the rabbit anti-interferon was 15,000—30,000 IFU-NU/ml. All immunoglobulins were isolated by 50% ammonium sulphate precipitation, followed by a dialysis versus phosphate buffer saline (PBS), pH 7.2.

Chemicals. CNBr was from Fluka (stored at −20°C). Sodium dodecylsulphate (SDS), specially pure for electrophorese, was purchased through British Drug House (BDH). Soyabean Trypsin Inhibitor (STI) and L-Lysine were obtained from Sigma. Sepharose 4B, CNBr-activated Sepharose 4B, CH-activated Sepharose 4B, and Epoxy-activated Sepharose 6B were all purchased from Pharmacia (Denmark).

Binding procedures. The covalent binding of the immunoglobulins to Sepharose 4B was done as previously described by K. Berg in Scand. J. Immunolog., 6, 77—86, (1977). Only 80—85% of the immunoglobulins were deliberately bound.

Protein determinations were made by a modification of the Lowry procedure (Berg K., Sequential Antibody Affinity Chromatography of Human Leukocyte Interferon, Scand. J. Immunol., 6, 77—86 (1977) which permitted detection of 1—2 µg/ml as the lowest level of proteins detectable (using an LKB Calculation Absorptioner Ultralab System). Crystalline bovine serum albumin was used as a standard protein. To determine the protein concentration of the purified interferon (1—5 µg in total) the following procedure was adopted: SDS was added to a final concentration of 0.1%. The lyophilized protein sample was further examined on an SDS-polyacrylamide gel electrophorese (SDS PAGE, see later), subsequent to a dialysis versus distilled water. The intensity of the stained protein bands was compared with known standards in different amounts (see later, under SDS PAGE), and the total amounts of proteins were estimated. The deviations were 5—10%, with the lowest detectable level of proteins being 0.1 µg (in total). The results from this method will serve as a rough estimate, rather than as an actual measurement.

Affinity chromatographies were performed at 4°C. The gel suspensions were degassed before packed into the columns. Packing was performed by washing with 3—5 bed volumes of loading buffer, using a peristaltic pump. Samples (100 µl) for interferon titrations were taken from either pools or individual fractions and titrated on the same day or frozen in plastic tubes (−20°C) and titrated later. The dilutions were made in medium (incl. 10% calf serum).

Antibody affinity chromatography was essentially done as described by Berg (Sequential Antibody Affinity Chromatography of Human Leukocyte Interferon, Scand. J. Immunol., 6, 77—86 (1977)). As loading buffer was used 0.1 M NaOA/0.3 M NaCl at pH 7.2 (flow rate 40 ml/h). Stepwise elution was performed with 0.1 M HOAc/0.3 M NaCl including a minute amount of citric acid (enough to keep the pH firmly at 2.4). When not operated, the column was stored at 4°C in PBS 1 M NaCl including Penicillin, Streptomycin, Gentamycin and Chloramphenical (1% of each). Before using the column for purification purposes, it was first washed with 100 ml of loading buffer followed by 10 ml of eluting buffer and finally equilibrated with 20—30 ml of loading buffer. This washing-cycle was necessary to avoid "spontaneous" proteins, especially when working with interferon of specific activities above $10^7$ IFU/mg proteins. The plastic tubes used for collecting the interferon eluate were pre-wetted with 100 µl of 1% SDS.

SDS PAGE. The purified, concentrated interferon preparations were analyzed for polypeptides components on SDS PAGE slab gels using 20 cm long separating gels, 0.75 mm thick (Bio Rad model 221: Dual vertical slab gel electrophoresis cell) and 7—10 cm long stacking gel. Exponential gradient gels of about 9—22% polyacrylamide were prepared by mixing 11 ml 22% acrylamide solution with about 32 ml 9% solution in a simple, ice-cooled gradient-device, as described in Knight, E., Interferon: Purification and initial characterization from human diploid cells. Proc. natn. Acad. Sci. USA 73, 520—523 (1976). The discontinuous buffer system, as described by Laenomli (Laenomli, U.K., Cleavage of Structural Proteins During Assembly of the Head of Bacteriophage T4, Nature 227, 680—685 (1970)) was used. The gel was pre-cooled for 2 h (10°C) before starting the actual electrophorese which was performed overnight (10°C) at constant effect (LKB power supply), starting out with 10 mA (and about 20 V). Samples to be analyzed were dissolved (or diluted) in 0.1 M Tris, HCl (pH 6.8) 2.5% SDS and 5% glucose including a tracking dye (sample buffer). The gel was stained in Comassie Blue (1.25 mg/ml in 50% methanol, 40% $H_2O$ and 10% acetic acid), without prior fixation, for 15 minutes at room temperature under constant rocking, and destained in 7% acetic acid (5% methanol). The gels were dried on paper of a good quality (for example, Whatman Chromatographic paper (17 mm)) under heat and vacuum using a gel dryer (Bio Rad, gel slab dryer, model 224). Solutions of five different molecular markers, from 0.1 µg to 10 µg of each marker per 20 µl, — Lactalbumin (14,400 Daltons); Soyabean Trypsin Inhibitor (20,100 Daltons); Carbonic Anhydrase (30,000 Daltons); Ovalbumin (43,000 Daltons); Bovine Serum Albumin (67,000 Daltons); Phosphorylase (94,000 Daltons) (obtained as an electrophoresis calibration kit (Pharmacia, Denmark)) — were subjected to SDS PAGE and stained. It should be noted that molecular weights assessed in this manner are subject to experimental accuracy of about ±200 Daltons. The stained protein bands were compared with the corresponding bands obtained from a parallel SDS PAGE of a purified interferon preparation and the total concentration of interferon proteins was estimated. For obtaining a biological profile from an SDS PAGE, the part of the gel intended for interferon determination, was cut from the remainder gel and kept at 4°C (in a humidified box) on a glass plate. The main part of the gel was stained for 15 minutes; after destaining for additionally 3—5 minutes, weak bands were clearly seen on a blue background, whereby the precise location of the protein bands corresponding to 14,000 and 30,000 Daltons could be established. The unstained part of the gel was cut, so it only contained proteins between 14,000 and 30,000 Daltons and was further subdivided in 1 mm pieces by sharp knives. The interferon from these slices was eluted with 0.5 ml 0.1 M SDS subsequent to a complete mincing by means of a teflon rod. After 5 h at room temperature (rocking) the interferon activity of the supernatant was determined. The individual fractions were frozen at −20°C without any additives.

Experimental section
Preparation of pure human leukocyte and lymphoblastoid interferon proteins

Concentration of crude human leukocyte interferon. To 3 liters of crude human leucocyte interferon was added KSCN up to a concentration of 0.5 M at pH 7.2. The pH was lowered by addition of 1N HCl to 4.5 (magnetic stirring) whereby a protein precipitate containing the interferon (and part of the impurities) was obtained. The precipitate was dissolved in 150 ml of PBS (phosphate buffered saline, pH 7.2) including 1 M NaCl and 25% by volume of ethylene glycol and dialyzed thoroughly versus 3 times 2 liters of the same buffer at 4°C. The specific activity of the crude concentrated human leukocyte interferon (HuLeCIF) was 5—10×$10^3$ IFU/mg protein. The recovery was about 98%.

Concentration of crude Namalva interferon. To 1 liter of crude Namalva interferon, with a titer of about 8000 IFU/ml, was added KSCN up to a concentration of 0.5 M at pH 7.2. The pH was lowered by addition of 1N HCl to 4.5 (magnetic stirring) whereby a protein precipitate containing the interferon (and part of the impurities) was obtained. The precipitate was dissolved in 50 ml of PBS, pH 7.2, including 1 M NaCl and 25% by volume of ethylene glycol and dialyzed thoroughly versus 3 times 2 liters of the same buffer at 4°C. The specific activity of the crude concentrated Namalva interferon (NaCIF) was 10—12×$10^3$ IFU/mg protein. The recovery was about 98%.

Gel filtration. A 100 cm long column (2.6 cm in diameter, Pharmacia K 2.6/100) was packed with ultrogel AcA 5/4 (LKB Denmark) in PBS containing 1 M NaCl and 25% by volume of ethylene glycol at 4°C (pH 7.2). After washing the column with 3 bed volumes of buffer, the column was stabilized. 10—15 ml of HuLeCIF (prepared as described above in 25% by volume of ethylene glycol, 1 M NaCl in BPS, pH 7.4) were loaded to the column, and the column was "eluted" with the loading buffer, the fractions being assayed for interferon activity. The interferon-containing fractions were pooled, and about 95% of the original interferon activity was recovered. The specific activity of the gelfiltered human leukocyte interferon-containing eluates was close to 1,000,000 IFU/mg protein, corresponding to a purification factor of 200. As determined by means of molecular markers, the molecular weight of the interferon corresponds to a range of 10,000—20,000 Daltons. Titrations of individual fraction revealed only one broad peak, with a maximum at 18,000 Daltons.

In the same manner as described above, 10 ml of NaCIF (prepared as described above in 25% by volume of ethylene glycol, 1M NaCl in PBS, pH 7.4) were loaded to the column, and the "elution" was performed in the same manner as described above. The recovery was about 90%. The specific activity of the gelfiltered Namalva interferon-containing eluate was close to 1,000,000 IFU/mg protein, corresponding to a purification factor of 100. As determined by means of molecular markers, the molecular weight of the interferon corresponds to a range of 10,000—20,000 Daltons. Titrations of the individual fractions revealed a broad peak, with a maximum at 18,000 Daltons.

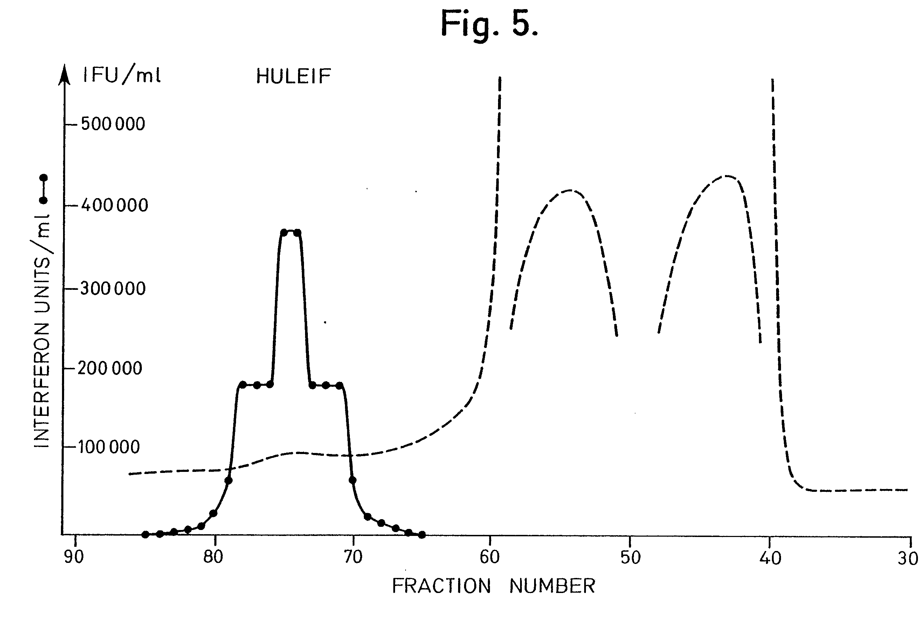

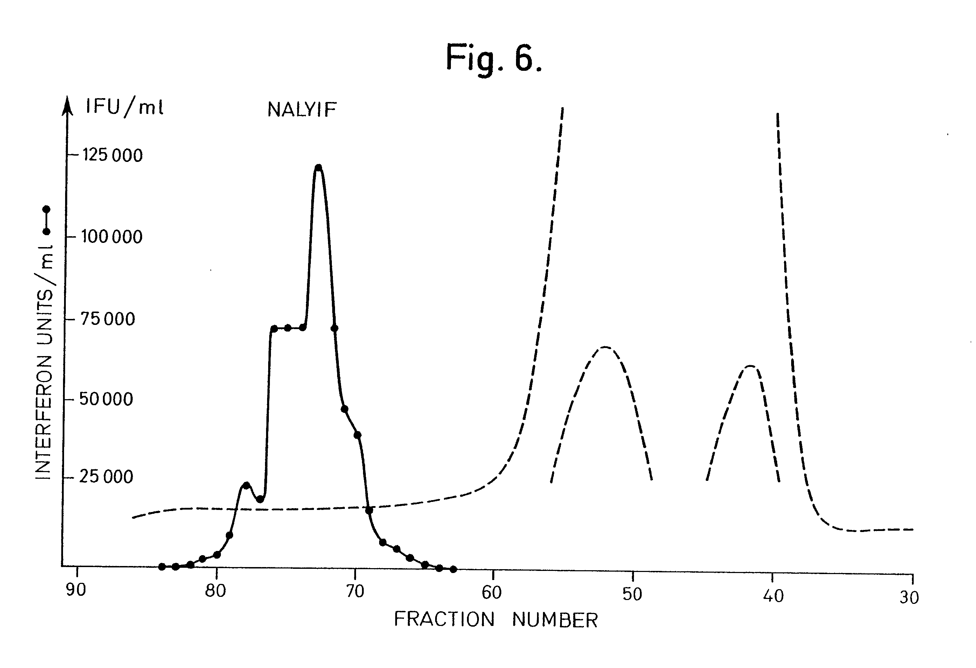

The gel filtration curves for the above-described gel filtration of HuLeCIF and NaCIF are shown in Figs. 5 and 6, respectively, and "HULEIF" indicates the human leukocyte interferon, whereas "NAYLYIF" indicates the Namalva (lymphoblastoid) interferon. It is clearly seen that the interferon activity is effectively separated from the major part of the proteins.

Blue Dextran chromatography. The gel-filtered human leukocyte interferon solution, obtained as described above, was exhaustively dialyzed against 200 volumes of 20 mM PB, pH 7.2 at 4°C. The dialysis was performed twice, the total dialysis time being about 24 hours. The dialyzed solution (25 ml, containing 1.8×$10^6$ IFU) was loaded on a column of Blue Dextran-Sepharose 4B. The diameter of the column was 1 cm, and the length of the column was 10 cm. The column was pre-washed with 200—300 ml of 20 mM PB (phosphate buffer) at pH 7.4. The dialyzed interferon preparation was loaded to the equilibrated column, and the column was washed with 75 ml of PB. 4500 IFU was found in the wash. The column was eluted with 0.6 M NaCl, 20 mM PB, pH 7.2 whereby more than 95% of the interferon activity was recovered in 6 ml of eluate, as determined by interferon titration.

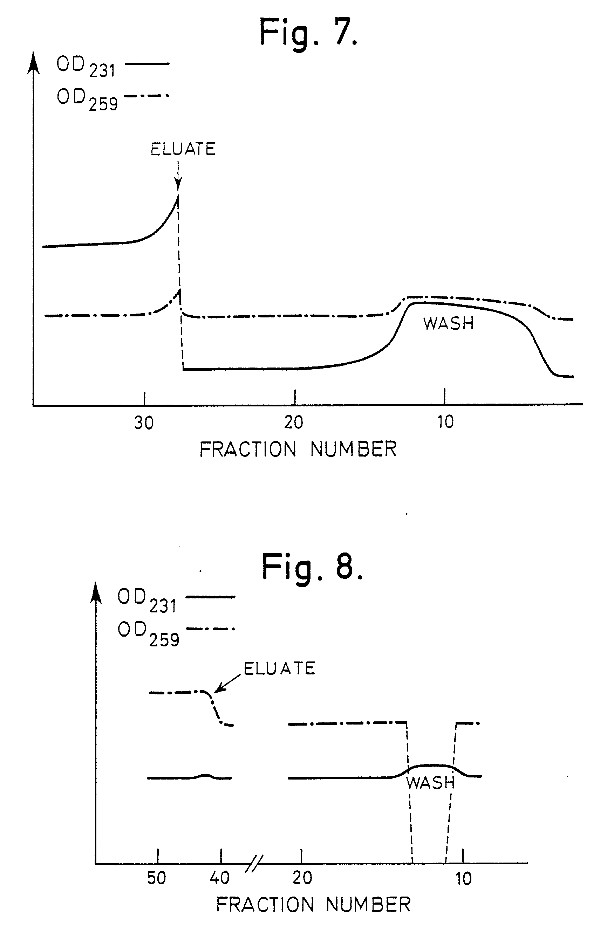

In exactly the same manner, the above-mentioned gel-filtered Namalva interferon solution was exhaustively dialyzed and thereafter subjected to Blue Dextran chromatography. The input in the Blue Dextran chromatography was 1,600,000 IFU. The wash consisted of 70,000 IFU in 50 ml. The eluate was obtained by means of 0.6 M NaCl in PB (pH 7.2). The Blue Dextran chromatography of Namalva interferon is illustrated in Fig. 7. The fibroblast part of the Namalva interferon was not eluted from the column under the above conditions, but is expected to be eluted using, e.g., 25% ethylene glycol in 1 M NaCl, pH 7.2.

The above-mentioned Blue Dextran column was a column of Blue Dextran (Cibracon Blue F3GA immobilized on Dextran 2000 (molecular weight 2 millions)) coupled to cyanogen bromide-activated agarose (Sepharose 4B). Thus, the more complete designation of the column is Blue-Dextran-Sepharose 4B. This type of column is described by Bollin et al., *loc. cit.* after elution, the column was cleaned by elution with 25—30 ml 25% ethylene glycol, 1.5 M NaCl in 20 mM PB. The column was stored in this buffer at 4°C when not in use. As mentioned above, the loading conditions could also involve the use of hydrophobic reagents, such as alcohols in various amounts (0—50%).

The 0.6 M NaCl eluates from the Blue Dextran chromatography show a specific activity of 70×$10^6$ IFU/mg of protein, both for the human leukocyte interferon and for the Namalva interferon. Thus, these are candidates for parenteral administration in human beings for therapeutic purposes and, in this regard, are much more pure preparations than the commonly used PIF preparations. For this use, the eluates are stabilized with physiologically acceptable stabilizers such as described further above, for example 1% of human albumin.

For further purification and for preparation of pure interferon, the eluates from the Blue Dextran column are directly transferred to an antibody affinity chromatography column. In the most advantageous embodiment, the antibody affinity chromatography column is combined with the Blue Dextran column in a "tandem system" as described below:

Tandem Affinity Chromatography. Instead of eluting the Blue Dextran column as described above, the Blue Dextran column is combined with the equilibrated antibody column prior to the elution, by connecting the outlet of the Blue Dextran column with the inlet of the antibody column. In this manner, the eluate from the Blue Dextran column is immediately "caught" by the antibody column. This combination makes use of the fact that the elution conditions (0.6 M NaCl, 20 mM PB, pH 7.2) can be used as loading conditions of the antibody column. After the elution/loading using 20 ml of the eluate/"loading buffer" (this "loading buffer", of course, at the same time contains the interferon eluted from the Blue Dextran column), the two columns are disconnected, and the antibody column is washed further before eluted as described above. The human leukocyte interferon eluate from the antibody column contains pure interferon proteins showing a specific activity of more than $10^9$ IFU/mg of protein (as assessed by the determination method discussed above). For stabilization of the pure interferon proteins, the tubes in which the eluate from the antibody column is collected (fraction size 2 ml) have been pre-wetted with 100 µl of 1% SDS each. After pooling of interferon-containing eluate, additional SDS is added up to a total concentration of 0.1% by weight.

The pooled interferon-containing eluates stabilized with 0.1% SDS are transferred to a 20 ml stainless steel tube pre-cooled to 0°C in an ice bath. After 15 minutes, a precipitate is formed. The precipitate is isolated by centrifugation at 20,000 rpm at 4°C for 20 minutes. The supernatant is discarded (no interferon activity), and the precipitate is redissolved in 4 ml of 8 M urea and transferred to a Millipore concentration cell, size 8 ml, filter 10,000 molecular weight cut, and concentrated to about 100 µl at room temperature. Thereafter, additional 4 ml 4 M urea (p.a.) was added to the concentrate, and the solution was concentrated to about 100 µl at room temperature. 1—3 ml of distilled water was added, and the solution was concentrated again to a volume of 20 µl and mixed with 20 µl SDS sample electrophoresis buffer. 20 µl of the resulting solution was used for characterization as described in the section "SDS PAGE" below.

The above-mentioned antibody affinity chromatography column had been prepared in accordance with "Binding Procedures" using non-monospecific anti-PIF which had been adbsorbed as follows: a total amount of $10^6$ IFU-NU of anti-interferon immunoglobulins (corresponding to 4 ml sheep anti-interferon serum) was absorbed three times on a 150 ml column of human serum bound to Sepharose 4B followed by 4 absorptions on a CIF-epoxy Sepharose column and 2 absorptions on a CIF CH-activated Sepharose 4B as described in the below section "Absorption of Anti interferon" and in Scand. J. Immunol. 8, 429—436 (1978). Finally, the immunoglobulins had been asborbed on a poly-L-lysine-Sepharose column (once) and on a Soyabean Trypsin Inhibitor-Sepharose column (twice).

The eluate from the Blue Dextran chromatography of Namalva interferon was divided in two portions. One portion was used for SDS PAGE electrophoresis as described below. 250,000 IFU were loaded to the absorbed antibody column as shown in Fig. 8. No interferon was found in the wash. The interferon was eluted as usual by lowering pH to 2.4, and 235,000 IFU (collected in the presence of 0.1% SDS) were recovered. This eluate was concentrated as described above and further examined in SDS PAGE.

SDS PAGE. The SDS PAGE electrophoresis was performed as described under "MATERIALS AND METHODS" above. The stained slab of the electrophoresis of the pure human leukocyte interferon proteins is shown in Fig. 1. Fig. 2 shows, schematically, the stained slab from another experiment, together with the corresponding interferon activity eluted from an unstained parallel gel strip. The striking reproducibility between the two experiments appears from the two Figures, the difference between 20,100 and 20,180 being within the experimental accuracy. As mentioned previously, the biological peaks coincide exactly with the proteins.

From Fig. 1, it appears that the interferon preparation is complete pure by SDS PAGE. There is no other protein band whatsoever visible.

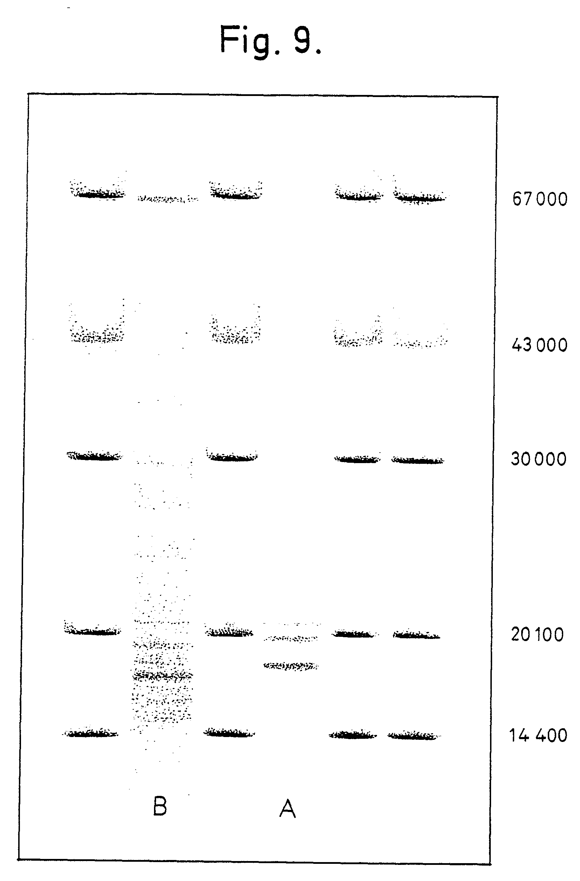

Fig. 9 shows the stained slab from the SDS PAGE (load $0.9 \times 10^6$ IFU), of the pure Namalva interferon proteins (A), and of the eluate from the Blue Dextran column (B). By comparison with Fig. 1, it will be noted that the bands of the pure Namalva interferon are essentially identical with the bands of pure human leukocyte interferon applied in the same amount.

Establishment of hybridoma cells with activity directed against interferon

3 female Balb/c mice, age two months, were immunized with human leukocyte interferon in the following way:

The first injection (40,000 IFU) was performed subcutaneously in the back of each mouse. The immunization was continued every week with subcutaneous injection of 70,000 IFU. The last injection was given intravenously the 9th week (mouse 1) and 10th week (mouses 2 and 3), respectively.

The development of anti-interferon was determined on serum samples from the mice, using the interferon neutralization test. As a laboratory check of the interferon neutralization test system, an internal anti-interferon IgG preparation (raised by injecting rabbits with partially purified human leukocyte interferon preparations) was, as usually, included. The serum samples from the mice showed no anti-interferon activity the first six weeks. Thereafter, distinct anti-interferon activity was found:

TABLE I
IFU-NU per ml

|  | 7th week | 8th week | 9th week | 10th week |
|---|---|---|---|---|
| mouse 1 | 160 | 160 | 120 | — |
| mouse 2 | 200 | 1280 | 2500 | 1200—2500 |
| mouse 3 | 80 | 40 | 40 | 5—10 |

The mice were killed by breaking their necks two to four days after the last injection, and their spleens were removed under sterile conditions. After homogenization of each spleen in PBS, the homogenized cell suspension was transferred to centrifuge tubes and centrifugated for 5 minutes at 170 g at 4°C. The cells were resuspended in PBS, and after a second centrifugation, they were resuspended in serum-free DMEM (about 0.5 ml per spleen). The total amount of cells was $10^8$ (mouse 1), $0.8 \times 10^8$ (mouse 2), and $0.8 \times 10^8$ (mouse 3). The viability was around 85—90%.

By treatment with polyethylene glycol in the manner described below, the spleen cell suspension from each mouse was fused with $10^7$X63Ag8 (HPRT-) myeloma cells in the following manner: $10^8$ mouse spleen cells and $10^7$ 8-azaguanin-resistent myeloma cells (X63Ag8; NSI/1Ag 4-1; SP 2/0-Ag 14) were mixed in a 50 ml conical plastic centrifuge tube (Falcon 2070). The tube was filled up with serum-free DMEM and centrifugated for 10 minutes at 170—200 g and 4°C. The supernatant was carefully removed, and at 37°C, a total of 0.7 ml of 50% polyethylene glycol solution having a temperature of 37°C was added dropwise over a period of 1 minute with gentle rotation. After incubation for 90 seconds at 37°C, 15 ml of warm serum-free DMEM were added very slowly (in the course of 1—2 minutes). Thereafter, the mixture was centrifugated for 10 minutes at 200 g, and the cell pellet was resuspended in 50 ml complete DMEM-FCS for seeding in Costar trays.

From each of the fusions, 48 cultures, each of 1 ml, were seeded in Costar trays (2 trays, each with 24 holes per spleen=48 cultures per mouse). After 10—15 days, growth was noted in 21 cultures (mouse 1), 0 cultures (mouse 2) and (after further seeding out) 150 cultures (mouse 3).

The cells were transferred to 5 ml cultures in 25 ml NUNC bottles which, like the Costar trays, contained a "feeder layer" of macrophages. On shift of medium, the supernatants were obtained, and from these dense cultures, cells were frozen in liquid nitrogen.

The supernatants of the individual cultures from mouse 1 were subjected to detection of positive clones using the interferon neutralization test. In this manner, one positive clone was found, although with a very low titer (about 2—3 IFU-NU per ml).

Production of anti-interferon by means of pure interferon proteins (pure by SDS PAGE)

The eluate from the above-described tandem affinity chromatography, as characterized by SDS PAGE, was used for immunization of rabbits as follows:

About 1,000,000 IFU units were concentrated to about 1 ml and dialyzed against PBS at 10°C overnight. Two rabbits were injected subcutaneously with each 1,000,000 IFU prepared in this manner. The injection was repeated each second week. The development of antibodies appears from Table II:

TABLE II
Neutralization units (IFU-NU)

3706

| | | | | | Freund's adjuvant | | | | |
|---|---|---|---|---|---|---|---|---|---|
| Rabbit I | **) | 1 | 3 | 5 | 7 | 9 | 11 | 13 | 15 |
| | ***) | 0 | 0 | 2 | 100 | 2000 | ND*) | 20,000 | 20,000 |

| | | Freund's adjuvant | | | | | |
|---|---|---|---|---|---|---|---|
| Rabbit I | **) | 17 | 19 | 21 | 23 | 25 | 27 |
| | ***) | 20,000 | 800,000 | 600,000 | 500,000 | 600,000 | 600,000 |

| | | | | | Freund's adjuvant | | | | |
|---|---|---|---|---|---|---|---|---|---|
| Rabbit II | **) | 1 | 3 | 5 | 7 | 9 | 11 | 13 | 15 |
| | ***) | 0 | 0 | 0 | 20 | 500 | ND*) | 20,000 | 10,000 |

| | | Freund's adjuvant | | | | | |
|---|---|---|---|---|---|---|---|
| Rabbit II | **) | 17 | 19 | 21 | 23 | 25 | 27 |
| | ***) | 8000 | 100,000 | 150,000 | 200,000 | 180,000 | 185,000 |

*) not determined
**) week
***) antiinterferon titers (IFU-NU/ml)

Production of anti-interferon by means of pure stained interferon proteins cut out from an SDS PAGE

The immunization was performed according to the protocol explained on page 11 of EP—A—0018218, using the minced interferon-containing (and partially washed and destained) gel suspension directly as the immunogenic preparation. One rabbit (III) was immunized with the 18,400±200 Daltons species, and another rabbit (IV) was immunized with the 20,100±200 Daltons species (died after week 15). Good results were obtained, vide Table III:

TABLE III
Neutralization units

| | | | | | | | Freund's adjuvant | |
|---|---|---|---|---|---|---|---|---|
| Rabbit III | week | 1 | 3 | 5 | 7 | 9 | 11 | 15 |
| (18,400 Daltons species) | IFU-NU/ml | 0 | 0 | 0 | 1—2 | 2 | 2 | 200 |
| | | | | | | | Freund's adjuvant | |
| Rabbit IV | week | 1 | 3 | 5 | 7 | 9 | 11 | 15 |
| (20,100 Daltons species) | IFU-NU/ml | 0 | 0 | 0 | 0 | 1 | 2 | 200 |

Antigenicity of the 18,400 Daltons species versus the 20,100 daltons species and vice versa

In order to show that the antigenic determinants of the above-mentioned two species are identical, the following cross-neutralization experiments were performed:

13

Interferon protein was eluted from the 18,400±200 Daltons species SDS PAGE band and the 20,100±200 Daltons species SDS PAGE band in the manner described above, and solutions containing 5—10 IFU of the two species were prepared. Solutions of anti-interferon from the two species, prepared in rabbits as described above, were diluted to contain 20 IFU-NU in total/ml. Aliquots (1 ml) of pure interferon species containing 10 IFU of the 18,400 Daltons interferon species and 10 IFU of the 20,100 Daltons interferon species, respectively, were mixed with 1 ml solution of the 18,400 Daltons species anti-interferon and 1 ml solution of the 20,100 Daltons species anti-interferon, respectively, in all possible permutations, that is, the anti-interferon of each species was mixed with the interferon of both species separately. After 1 hour at 37°C, any remaining interferon activity was determined by performing the usual interferon titration (vide "Materials and Methods" above). No interferon activity was found. Thus, when mixing the 18,400±200 Daltons species and the 20,100±200 Daltons species, respectively, with each of the anti-18,400±200 Daltons species, and the anti-20,100±200 Daltons species, separately, and *vice versa*, no interferon was detected, in other words, a complete neutralization had occurred. Therefore, it can be concluded that the two interferon species exhibit the same antigenic determinants. This implies that the anti-18,400±200 Daltons species will be useable as a monospecific antibody for purification of both interferon species, and the same applies for the anti-20,100±200 Daltons species, and for a mixture of the two species. Further experiments performed in the same manner showed that each of the six biological peaks was completely neutralized by each of the antisera raised against the two major species.

It is highly likely that the two major species isolated from the Namalva SDS PAGE will give the same result, in other words, that they also cross-react and show identity to HuLeIf in terms of antigenicity. (HuLeIF 18,400±200 being identical to Namalva 18,400±200, both with respect to antigenicity and molecular weight, and HuLeIF 20,100±200 being similarly identical with Namalva 20,100±200).

Biological effects of the pure interferon proteins

Antiviral activity

The antiviral activity of each of the six stained protein bands shown in Fig. 3 was determined. The gel was loaded in two slots, both of which were stained. The stained bands in one of the slots is shown at A in Fig. 3. The other slot was then briefly destained (in 50% methanol, 45% $H_2O_2$, 5% acetic acid), the exact location of the interferon proteins in the wet gel was recorded, and the gel was rinsed in water and was thereafter sliced as shown at B in Fig. 3. The number of gel slices is indicated at C in Fig. 3. In this manner, each interferon protein band was exactly cut out of the gel, without being mixed with the adjacent one. Each slice was eluted in the same manner as described in the section "Materials and Methods", and the biological profile shown in Fig. 3 was constructed using the usual interferon titration described in "Material and Methods". The neutralizing activity of each of the six species cut out and eluted from the SDS PAGE was checked against anti-leukocyte interferon, and it was found that all of the species were completely neutralized by the same antiserum. The recovery of interferon in Fig. 3 was rather low (20%) compared with normal "SDS PAGE elution" without pre-staining (except for the 18,400±200 Daltons species), which indicates that the biological activity of most of the interferon species was selectively destroyed compared with the antigenicity. In the neutralization tests against anti-leukocyte interferon, the interferon proteins "eluted from Fig. 3" were able to neutralize the anti-leukocyte interferon 3—5 times more effectively than native (crude) human leukocyte interferon, calculated on interferon activity basis, which indicates a selective destruction of determinants responsible for the biological activity.

Non-antiviral effects

The non-antiviral effects of the pure human leukocyte interferon species were checked in 3 systems:

1) Anti-cellular activity

The anticellular activity of the pure interferon proteins was investigated by incubating Daudi cells with 1:1000 dilutions (in medium) of pure interferon proteins obtained from the eluted SDS PAGE fractions as shown in Fig. 2, by ascertaining the relative depression of Tritium labelled Thymidine (I. Heron and K. Berg, The actions of interferon are potentiated at elevated temperature, Nature, *274*, 508—510 (1978)) compared to controls without interferon (Fig. 2, upper part, where "% G-I" designates % growth inhibition). As can be clearly seen, the "anticellular curve" follows the antiviral curve very strictly. This proves that all the five species of pure native human leukocyte interferon contain both the antiviral activity and the anticellular activity. The peak size of the different "anticellular peaks" does not vary linearly with the corresponding size of the "interferon peaks", which probably reflects the sensitivity of the Daudi cell system (J. Hilfenhaus, H. Damm, H. E. Karges and K. E. Manthey, Growth inhibition of human lymphoblastoid Daudi cells in vitro by interferon preparations, Arch. Virol. *51*, 87—97 (1976). The small interferon peak at 19,500 Daltons gave no rise to a corresponding peak in the anticellular curve. At a 10-fold lower dilution (1:100), however, a small but distinct peak of anticellular activity was also observed (not shown).

2) The expression of major histocompatibility antigen (MHC) on lymphocytes and monocyte

The selective increase in $\beta_2$-associated MHC (major histocompatibility antigen) expression was observed using partially purified human leukocyte interferon, such as described by I. Heron, M. Hokland & K. Berg (1978), "Enhanced expression of $\beta_2$ microglobulin and HLA on human lymphoid cells by

14

interferon", Proc. Natl. Acad. Sci. *75*: 6215—6222 (referred to below as PNAS *75*). Each of the two major human leukocyte interferon species (18,400 and 20,100 Daltons, vide Fig. 1), was assayed in doses around 100 IFU per ml culture medium. The above-mentioned effect was found using these pure molecular species, whereas eluates from gel slices outside the regions where antiviral activity was recorded had no effect. It has, thus, been proved that the effect of selective enhancement of MHC antigen expression on lymphoid cells is an inherent feature of the interferon molecules.

3) The potentiation of the Natural Killer cell system (NK system)

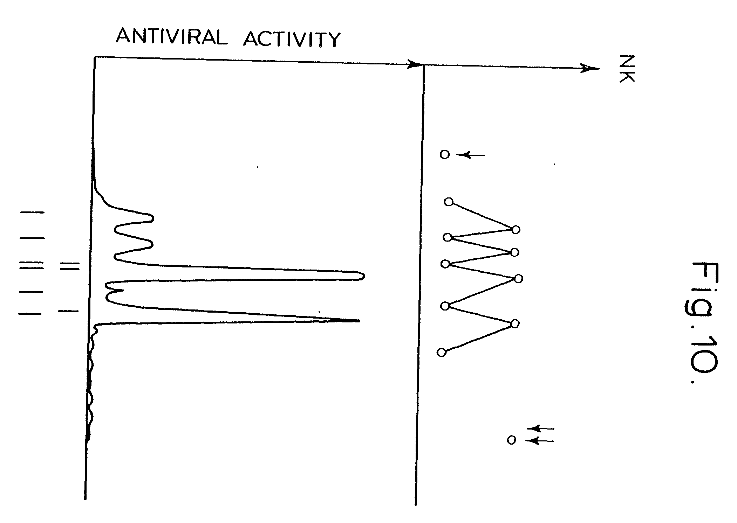

Fig. 10 shows the antiviral profile (as assessed on an SDS PAGE in the same manner as described in connection with Fig. 2). Each of the species from the gel was assessed for NK enhancing activity, using the method described in PNAS *75*. Fractions that have antiviral activity as shown in the lower curve gave increased NK, such as illustrated in the upper curve, whereas "base line" fractions did not. One arrow indicates only saline added as a negative control, and two arrows indicate partially purified human leukocyte interferon (PIF) used as a positive control. Around 100 IFU antiviral units of each interferon preparation was added per ml.

**Claims**

1. Human Le form interferon proteins consisting of six interferon components which under the SDS PAGE and staining conditions defined herein at a total interferon load of $0.9\times10^6$ IFU resolve to show two major sharp stained protein bands having antiviral interferon activity at 18,400 and 20,100 Daltons, respectively, and a minor stained protein band having antiviral interferon activity between 20,300 and 20,400 Daltons, together with smaller peaks of antiviral interferon activity at 19,500, 21,130 and 23,440 Daltons (said Dalton molecular weights being subjected to an experimental accuracy $\pm200$ Daltons), the stained protein regions of said SDS PAGE acrylamide gradient being essentially only stained interferon proteins.

2. Human Le form interferon proteins as claimed in claim 1 further characterised in that under the SDS PAGE and staining conditions defined herein at a total interferon load of $3.8\times10^6$ IFU they resolve to show six stained protein bands having antiviral interferon activity, viz. strong bands at 18,410 Daltons and 20,180 Daltons respectively, a medium-strong band at 20,420 Daltons and just visible bands at 19,500 Daltons, 21,130 Daltons and 23,440 Daltons respectively (said Dalton molecular weights being subject to an experimental accuracy of $\pm200$ Daltons), the peaks of antiviral activity coinciding exactly with the stained protein bands, the stained protein regions of said SDS PAGE acrylamide gradient being essentially only stained interferon proteins.

3. Each individual protein having antiviral interferon activity which is a component of the human Le form interferon proteins claimed in claim 1 or claim 2.

4. A protein having antiviral interferon activity as claimed in claim 3 appearing at 18,400±200 Daltons.

5. A protein having antiviral interferon activity as claimed in claim 3 appearing at 20,100±200 Daltons.

6. A protein having antiviral interferon activity as claimed in claim 3 appearing between 20,300 and 20,400 Daltons (said Dalton molecular weights being subject to an experimental accuracy of ±200 Daltons).

7. A protein having antiviral interferon activity as claimed in claim 3 appearing at 19,500±200 Daltons.

8. A protein having antiviral interferon activity as claimed in claim 3 appearing at 21,130±200 Daltons.

9. A protein having antiviral interferon activity as claimed in claim 3 appearing at 23,400±200 Daltons.

10. A formulation comprising a protein or proteins as claimed in any of the preceding claims, said formulation being adapted for administration to human beings or animals for prophylactic, therapeutic, or immunization effect.

11. A formulation as claimed in claim 10 adapted for parenteral, intranasal, or topical administration.

12. A formulation as claimed in claim 10 in the form of a stabilized aqueous solution.

13. A formulation as claimed in claim 12 in which the stabilizer is a protein or combination of proteins which is non-toxic and non-immunogenic in human beings.

14. A formulation as claimed in claim 13 in which the stabilizer is selected from the group consisting of human serum proteins and fractions thereof, and human albumin.

15. A formulation as claimed in any of claims 10—14 in which the concentration of the pure interferon is in the range corresponding to 1—20 million IFU per ml.

16. A formulation as claimed in claim 12 for immunizing animals, in which the stabilizer is SDS.

17. A formulation as claimed in claim 16 which is buffered with PBS with a pH of about 7.2.

18. A formulation as claimed in claim 16 or 17 which additionally contains an adjuvant.

19. A formulation as claimed in claim 18 in which the adjuvant is Freund's adjuvant.

20. An SDS complex of a protein or proteins as claimed in any of claims 1 to 9.

21. An SDS complex as claimed in claim 20 isolated in solid form.

22. A protein which shows interferon activity and which is able to bind antibodies raised in rabbits against any of the interferon proteins claimed in any of claims 1—9.

23. A method of producing human Le form interferon proteins as claimed in claim 1 or claim 2, comprising subjecting a solution containing human Le form interferon proteins to affinity chromatography

## EP 0 018 218 B1

such that said human Le form interferon proteins are selectively retained, and eluting said retained interferon proteins.

24. A method as claimed in claim 23 wherein the solution containing human Le form interferon proteins subjected to antibody affinity chromatography has a specific activity of more than $10^7$ IFU/mg protein, and is prepared by protein precipitation of human Le form interferon proteins from a solution thereof, subjecting a solution containing said precipitated proteins to gel filtration, further purifying the eluted fractions having interferon activity by dialysis, and subjecting the dialysed interferon proteins to ligand affinity chromatography.

25. A method as claimed in claim 24 wherein the protein precipitation step includes the addition of KSCN and the adjustment of pH to 4.5.

26. A method as claimed in claim 24 or claim 25 wherein the interferon fractions to be purified by dialysis contain essentially only proteins in the molecular weight range 10,000 to 20,000 Daltons as assessed by their elution behaviour at the gel filtration stage.

27. A method as claimed in claim 26 wherein the gel filtration is performed with a buffer solution which contains about 25% by volume of a glycol such as ethylene glycol, and has an ionic strength corresponding to 1M NaCl, pH about 7.2.

28. A method as claimed in any of claims 24 to 27 wherein the dialysis is performed exhaustively against 20 mM phosphate buffer.

29. A method as claimed in any of claims 24 to 28 wherein the ligand in the ligand chromatography is Cibacron F3GA, or another ligand capable of binding the human Le form interferon proteins according to the mechanism exerted by Cibacron F3GA.

30. A method as claimed in claim 29 wherein the Cibacron F3GA is matrix immobilized as Blue Dextran 2000, Blue Sepharose CL-68, or Blue Dextran coupled to Sepharose 4B.

31. A method as claimed in any of claims 23 to 30 wherein the antibodies used in the antibody affinity chromatography are raised against, or directed substantially only against, immunological determinants of human Le form interferon proteins as claimed in any of claims 1—9, and said antibodies are immobilized on a matrix.

32. A method as claimed in claim 23 wherein the antibodies used in the antibody affinity chromatography are raised against, or directed substantially only against, immunological determinants of human Le form interferon proteins as claimed in any of claims 1—9, said antibodies are immobilized on a matrix, and the solution applied to the antibody affinity matrix is a crude, unconcentrated interferon preparation, or a concentrated or partially purified preparation.

33. A method as claimed in claim 31 or claim 32 wherein the antibodies are first obtained by immunizing an immunizable animal with the 18,400±200, the 20,100±200, or a combination of the 18,400±200 and 20,100±200 Daltons human Le form interferon protein components of the proteins claimed in claim 1 or claim 2.

34. A method as claimed in any of claims 31—33 wherein the antibodies are covalently bound to the matrix.

35. A method as claimed in claim 34 wherein the matrix is a cross-linked agarose such as Sepharose 4B.

36. A method as claimed in any of claims 31—35 wherein the antibodies are substantially free of proteolytic enzymatic activity.

37. A method as claimed in claim 36 wherein the antibodies have been substantially freed from any proteolytic enzymatic activity by treatment with enzyme inhibitors or destructors.

38. A method as claimed in claim 37 wherein the antibodies have been treated with matrix immobilized enzyme inhibitors or destructors.

39. A method as claimed in claim 38 wherein the antibodies, prior to their covalent binding to the matrix, have been passed through a column of matrix immobilized poly-L-lysin and/or matrix immobilized Soyabean Trypsin inhibitor, and/or matrix immobilized kallikrein inactivator.

40. A method as claimed in any of claims 23—39 wherein the human Le form interferon of the solution subjected to antibody affinity chromatography is selected from the group consisting of human leukocyte interferons human lymphoblastoid interferon, and proteins as claimed in claim 22.

41. A method of preparing the human Le form interferon proteins as claimed in claims 3—9 comprising subjecting human Le form interferon proteins as claimed in claim 1 or claim 2 to the SDS PAGE conditions defined herein, thereby separating the said proteins according to their molecular weights, and recovering the individual interferon-active components from their relative positions in the PAGE gel.

**Patentansprüche**

1. Menschliche Le-Form-Interferonproteine, die aus 6 Interferon-Komponenten bestehen, welche sich unter den hier definierten SDS PAGE- und Färbebedingungen bei einer Gesamt-Interferonmenge von $0,9 \times 10^6$ IFU in zwei deutliche gefärbte Proteinhauptbanden von 18 400 bzw. 20 100 Dalton mit antiviraler Interferonaktivität und in eine gefärbte Proteinnebenbande von 20 300 bis 20 400 Dalton mit antiviraler Interferonaktivität und in kleinere Peaks von 19 500, 21 130 und 23 440 Dalton mit antiviraler Interferonaktivität trennen (wobei die experimentelle Genauigkeit dieser Daltonmolekulargewichte±200 Dalton ist)

16

# EP 0 018 218 B1

und die gefärbten Proteinbereiche des SDS PAGE Acrylamid-Gradienten im wesentlichen nur gefärbte Interferonproteine darstellen.

2. Menschliche Le-Form-Interferonproteine nach Anspruch 1, die weiter dadurch gekennzeichnet sind, daß sie sich unter den hier definierten SDS PAGE- und Färbebedingungen bei einer Gesamtinterferonmenge von $3,8\times10^6$ IFU in 6 gefärbte Proteinbanden mit antiviraler Interferonaktivität trennen, nämlich in starke Banden von 18 410 Dalton bzw. 20 180 Dalton, in eine mittelstarke Bande von 20 420 Dalton und in gerade noch sichtbare Banden von 19 500 Dalton, 21 130 Dalton bzw. 23 440 Dalton (wobei die experimentelle Genauigkeit dieser Daltonmolekulargewichte+200 Dalton ist), wobei die Peaks der antiviralen Aktivität genau mit den gefärbten Proteinbanden übereinstimmen und die gefärbten proteinbereiche des SDS PAGE Acrylamid-Gradienten im wesentlichen nur gefärbte Interferonproteine darstellen.

3. Jedes Einzelprotein mit antiviraler Interferonaktivität, das eine Komponente der in Anspruch 1 oder Anspruch 2 beanspruchten menschlichen Le-Form-Interferonproteine ist.

4. Protein mit antiviraler Interferonaktivität nach Anspruch 3, das 18 400±200 Dalton hat.

5. Protein mit antiviraler Interferonaktivität nach Anspruch 3, das 20 100±200 Dalton hat.

6. Protein mit antiviraler Interferonaktivität nach Anspruch 3, das zwischen 20 300 und 20 400 Dalton hat, wobei die experimentelle Genauigkeit dieser Daltonmolekulargewichte ±200 Dalton ist.

7. Protein mit antiviraler Interferonaktivtät nach Anspruch 3, das 19 500±200 Dalton hat.

8. Protein mit antiviraler Interferonaktivität nach Anspruch 3, das bei 21 130±200 Dalton hat.

9. Protein mit antiviraler Interferonaktivität nach Anspruch 3, das 23 400±200 Dalton hat.

10. Zusammensetzung, umfassend ein oder mehrere Proteine nach einem der vorstehenden Ansprüche, wobei die Zusammensetzung zur Applikation an Menschen oder Tieren für Prophylaxe, Therapie oder Immunisierung geeignet ist.

11. Zusammensetzung nach Anspruch 10, die für parenterale, intranasale oder örtliche Applikation geeignet ist.

12. Zusammensetzung nach Anspruch 10 in der Form einer stabilisierten wäßrigen Lösung.

13. Zusammensetzung nach Anspruch 12, in der der Stabilisator ein Protein oder eine Kombination von Proteinen ist, die nicht toxisch und nicht immunogen für Menschen ist.

14. Zusammensetzung nach Anspruch 13, in der der Stabilisator ein menschliches Serumprotein, eine Fraktion davon oder menschliches Albumin ist.

15. Zusammensetzung nach einem der Ansprüche 10 bis 14, in der die Konzentration des reinen Interferons im Bereich von 1 bis 20 Millionen IFU pro ml liegt.

16. Zusammensetzung nach Anspruch 12 zur Immunisierung von Tieren, in der der Stabilisator SDS ist.

17. Zusammensetzung nach Anspruch 16, die durch PBS bei einem pH-Wert von etwa 7,2 gepuffert ist.

18. Zusammensetzung nach Anspruch 16 oder 17, die ferner ein Adjuvans enthält.

19. Zusammensetzung nach Anspruch 18, in der das Adjuvans Freund's Adjuvans ist.

20. SDS-Komplex aus einem oder mehrerer Proteine eines der Ansprüche 1 bis 9.

21. SDS-Komplex nach Anspruch 20, der als Feststoff isoliert wird.

22. Protein, das Interferonaktivität zeigt und das Antikörper bilden kann, die in Kaninchen gegen eines der Interferon-Proteine nach einem der Ansprüche 1 bis 9 produziert wurden.

23. Verfahren zur Herstellung von menschlichen Le-Form-Interferonproteinen nach Anspruch 1 oder 2, dadurch gekennzeichnet, daß man eine Lösung von menschlichen Le-Form-Interferonproteinen einer Affinitätschromatographie unterwirft, so daß diese menschlichen Le-Form-Interferonproteine selektiv zurückgehalten werden und daß man diese Interferonproteine eluiert.

24. Verfahren nach Anspruch 23, dadurch gekennzeichnet, daß die Lösung die menschliche Le-Form-Interferonproteine enthält und die einer Antikörperaffinitätschromatographie unterworfen wird, eine spezifische Aktivität von mehr als $10^7$ IFU pro mg Protein hat und durch Proteinausfällung von menschlichen Le-Form-Interferonproteinen aus einer Lösung davon hergestellt wird, daß man eine Lösung, die die ausgefällten Proteine enthält, einer Gelfiltration unterwirft, die eluierten Fraktionen mit Interferon aktivität durch Dialyse weiter reinigt und die dialysierten Interferonproteine einer Liganden-Affinitätschromatographie unterwirft.

25. Verfahren nach Anspruch 24, dadurch gekennzeichnet, daß die Proteinausfällungsstufe die Zugabe von KSCN und das Einstellen des pH-Wertes auf 4,5 umfaßt.

26. Verfahren nach Anspruch 24 oder Anspruch 25, dadurch gekennzeichnet, daß die durch Dialyse zu reinigenden Interferonfraktionen im wesentlichen nur Proteine im Molekulargewichtsbereich von 10 000 bis 20 000 Dalton enthalten, wie durch ihr Eluierungsverhalten in der Gelfiltrationsstufe festgestellt.

27. Verfahren nach Anspruch 26, dadurch gekennzeichnet, daß die Gelfiltration mit einer Pufferlösung durchgeführt wird, die etwa 25 Vol.-% eines Glykols, wie Äthylenglykol, enthält und eine Ionenstärke entsprechend 1 M Na Cl und einen pH-Wert von etwa 7,2 hat.

28. Verfahren nach einem der Ansprüche 24 bis 27, dadurch gekennzeichnet, daß die Dialyse vollständig gegen 20 mM Phosphatpuffer durchgeführt wird.

29. Verfahren nach einem der Ansprüche 24 bis 28, dadurch gekennzeichnet, daß der Ligand in der Liganden-Chromatographie Cibacron F3GA ist oder ein anderer Ligand mit der Fähigkeit ist, menschliche Le-Form-Interferonproteine gemäß dem Mechanismus von Cibacron F3GA zu binden.

30. Verfahren nach Anspruch 29, dadurch gekennzeichnet, daß das Cibacron F3GA als Dextran Blau

# EP 0 018 218 B1

2000, Sepharose Blau CL-68 oder Dextran Blau, gekoppelt mit Sepharose 4B an einer Matrix immobilisiert ist.

31. Verfahren nach einem der Ansprüche 23 bis 30, dadurch gekennzeichnet, daß die Antikörper, die für die Antikörper-Affinitätschromatographie verwendet werden, gegen Immunologische Determinaten von menschlichen Le-Form-Interferonproteine nach einem der Ansprüche 1 bis 9 hergestellt oder im wesentlichen nur gegen diese gerichtet sind, und daß diese Antikörper an einer Matrix immobilisiert sind.

32. Verfahren nach Anspruch 23, dadurch gekennzeichnet, daß die Antikörper, die für die Antikörper-Affinitätschromatographie verwendet werden, gegen immunologische Determinanten von menschlichen Le-Form-Interferonproteinen nach einem der Ansprüche 1 bis 9 hergestellt oder im wesentlichen nur gegen diese gerichtet sind, wobei diese Antikörper an einer Matrixe immobilisiert sind, und die Lösung, die auf die Antikörper-Affinitätsmatrix aufgetragen wird, ein rohes, unkonzentriertes Interferonpräparat oder ein Konzentriertes oder teilgereinigtes Präparat ist.

33. Verfahren nach Anspruch 31 oder 32, dadurch gekennzeichnet, daß die Antikörper erst erzeugt werden durch Immunisieren eines immunisierbaren Tieres mit der 18 400±200, der 20 100±200 oder einer Kombination der 18 400±200 und 20 100±200 Dalton großen menschlichen Le-Form-Interferonprotein-Komponenten der in Anspruch 1 oder Anspruch 2 beanspruchten Proteine.

34. Verfahren nach einem der Ansprüche 31 bis 33, dadurch gekennzeichnet, daß die Antikörper kovalent an der Matrix gebunden sind.

35. Verfahren nach Anspruch 34, dadurch gekennzeichnet, daß die Matrix eine quervernetzte Agarose wie Sepharose 4B ist.

36. Verfahren nach einem der Ansprüche 31 bis 35, dadurch gekennzeichnet, daß die Antikörper im wesentlichen frei von proteolytischer enzymatischer Aktivität sind.

37. Verfahren nach Anspruch 36, dadurch gekennzeichnet, daß die Antikörper durch Behandlung mit Enzyminhibitoren oder Abbauern von im wesentlichen proteolytischer enzymatischer Aktivität befreit worden sind.

38. Verfahren nach Anspruch 37, dadurch gekennzeichnet, daß die Antikörper mit an einer Matrix immobilisierten Enzym-Inhibitoren oder Abbauern behandelt worden sind.

39. Verfahren nach Anspruch 38, dadurch gekennzeichnet, daß die Antikörper, bevor sie kovalent an die Matrix gebunden werden, durch eine Säule von an einer Matrix immobilisiertem Poly-L-Lysin und/oder an einer Matrix immobilisertem Soyabohnen-Trypsin-Inhibitor und/oder an einer Matrix immobilisertem Kallikrein-Inaktivator gegeben worden sind.

40. Verfahren nach einem der Ansprüche 23 bis 39, dadurch gekennzeichnet, daß das menschliche Le-Form-Interferon der Lösung, die einer Antikörper-Affinitätschromatographie unterworfen wird, ein menschliches Leukozyten-Interferon, ein menschliches Lymphoblasten-Interferon oder ein Protein nach Anspruch 22 ist.

41. Verfahren zur Herstellung von menschlichen Le-Form Interferonproteinen nach Anspruch 3 bis 9, dadurch gekennzeichnet, daß man menschliche Le-Form-Interferonproteine nach Anspruch 1 oder Anspruch 2 den hier definierten SDS PAGE-Bedingungen unterwirft, wobei die Proteine gemäß ihren Molekulargewichten getrennt werden und die einzelnen Interferonaktiven Komponenten von dem entsprechenden Teil des PAGE-Gels isoliert werden.

## Revendications

1. Protéines d'interféron sous form le d'être humain consistant en six composants d'interféron qui, en conditions SDS PAGE et de coloration définies ici à une charge totale d'interféron de $0,9 \times 10^6$ IFU se résolvent pour présenter deux bandes majeures fortement colorées de protéines ayant une activité antivirale d'interféron à 18.400 et 20.100 Daltons respectivement et une bande de protéines de coloration mineure ayant une activité antivirale d'interféron entre 20.300 et 20.400 Daltons, avec des pics plus petits d'activité antivirale d'interféron à 19.500, 21.130 et 23,440 Daltons (lesdits poids moléculaires en Daltons étant soumis à une précision expérimentale de ±200 Daltons), les régions des protéines colorées dudit gradient d'acrylamide SDS PAGE n'étant essentiellement que das protéines colorées d'interféron.

2. Protéines d'interféron sous forme Le d'être humain selon la revendication 1 caractérisées de plus en ce que dans les conditions SDS PAGE et de coloration définies ici à une charge totale d'interféron de $3,8 \times 10^6$ IFU, elles se résolvent pour présenter six bandes colorées de protéines ayant une activité antivirale d'interféron, c'est-à-dire des bandes fortes à 18.410 Daltons et 20.180 Daltons respectivement, une bande moyennement forte à 20.420 Daltons et des bandes juste visibles à 19.500 Daltons, 21.130 Daltons et 23.440 Daltons respectivement (lesdits poids moléculaires en Daltons étant soumis à une précision expérimentale de +200 Daltons), les pics de l'activité antivirale coïncidant exactement avec les bandes colorées de protéines, les régions colorées de protéines dudit gradient d'acrylamide SDS PAGE étant essentiellement uniquement des protéines colorées d'interféron.

3. Chaque protéine indivuelle ayant une activité antivirale d'interféron qui est un composant des protéines d'interféron sous form Le d'être humain selon la revendication 1 ou la revendication 2.

4. Protéine ayant une activité antivirale d'interféron selon la revendication 3 apparaissant à 18.400±200 Daltons.

5. Protéine ayant une activité antivirale d'interféron selon la revendication 3 apparaissant à 20.100±200 Daltons.

6. Protéine ayant une activité antivirale d'interféron selon la revendication 3 apparaissant entre 20.300 et 20.400 Daltons (lesdits poids moléculaire en Daltons étant soumis à une précision expérimentale de ±200 Daltons).

7. Protéine ayant une activité antivirale d'interféron selon la revendication 3 apparaissant à 19,500±200 Daltons.

8. Protéine ayant une activité antivirale d'interféron selon la revendication 3 apparaissant à 21.130±200 Daltons.

9. Protéine ayant une activité antivirale d'interféron selon la revendication 3 apparaissant à 23.400±200 Daltons.

10. Formule comprenant une protéine ou des protéines selon l'une quelconque des revendications précédentes, ladite formule étant adaptée à une administration à des êtres vivants ou des animaux pour un effet prophylactique, thérapeutique ou d'immunisation.

11. Formule selon la revendication 10 adaptée à une administration parentérale, intranasale ou topique.

12. Formule selon la revendication 10 sous la forme d'une solution aqueuse stabilisée.

13. Formule selon la revendication 12 dans laquelle le stabilisant est une protéine ou une combinaison de protéines qui est non toxique et non immunogène chez les êtres humains.

14. Formule selon la revendication 13 où le stabilisant est choisi dans le groupe consistant en protéines de sérum humain et leurs fractions, et albumine humaine.

15. Formule selon l'une quelconque des revendications 10—14 où la concentration de l'interféron pur est dans la plage correspondant à 1—20 millions IFU par ml.

16. Formule selon la revendication 12 pour immuniser des animaux, où le stabilisant est SDS.

17. Formule selon la revendication 16 qui est tamponée par PBS avec un pH d'environ 7,2.

18. Formule selon la revendication 16 ou 17 qui contient de plus un adjuvant.

19. Formule selon la revendication 18 où l'adjuvant est l'adjuvant de Freund.

20. Complexe SDS d'une protéine ou de protéines selon l'une quelconque des revendications 1 à 9.

21. Complexe SDS selon la revendication 20 isolé sous forme solide.

22. Protéine qui présente une activité d'interféron et qui peut lier des anticorps élevés chez des lapins contre toutes les protéines d'interféron selon l'une quelconque des revendications 1—9.

23. Méthode de production de protéines d'interféron sous forme Le d'être humain selon la revendication 1 ou la revendication 2, consistant à soumettre une solution contenant les protéines d'interféron sous forme Le d'être humain à une chromatographie par affinité de manière que lesdites protéines d'interféron sous forme Le d'être humain soient sélectivement retenues, et en éluant lesdites protéines retenues d'interféron.

24. Méthode selon la revendication 23 où la solution contenant les protéines d'interféron sous forme Le d'être humain soumises à une chromatographie par affinité d'anticorps a une activité spécifique de plus de $10^7$ IFU/mg protéines et est préparée par précipitation de protéines, des protéines d'interféron sous forme Le d'être humain de leur solution, en soumettant une solution contenant lesdites protéines précipitées à une filtration sur gel, en purifiant encore les fractions éluées ayant une activité d'interféron par dialyse et en soumettant les protéines dialysées d'interféron à une chromatographie par affinité à un ligand.

25. Méthode selon la revendication 24 où l'étape de précipitation des protéines comprend l'addition de KSCN et l'ajustement du pH à 4,5.

26. Méthode selon la revendication 24 ou 25 où les fractions d'interféron à purifier par dialyse ne contiennent essentiellement que des protéines dans la plage de poids moléculaire de 10.000 à 20.000 Daltons en déterminant par leur comportement d'élution au stade de la filtration sur gel.

27. Méthode selon la revendication 26 où la filtration sur gel est accomplie avec une solution tampon qui contient environ 25% en volume d'un glycol tel que l'éthylène glycol et a une force ionique correspondant à NaCl 1M, pH environ 7,2.

28. Méthode selon l'une quelconque des revendications 24 à 27 où la dialyse est accomplie de manière exhaustive contre un tampon de phosphate 20 mM.

29. Méthode selon l'une quelconque des revendications 24 à 28 où le ligand dans la chromatographie par ligand est Cibacron F3GA, ou autre ligand capable de lier les protéines d'interféron sous forme Le d'être humain selon le mécanisme exercé par Cibacron F3GA.

30. Méthode selon la revendication 29 où Cibacron F3GA est immobilisé sur matrice sous formé de Bleu Dextrane 2000, Bleu Sepharose CL-68 ou Bleu Dextrane couplé à Sepharose 4B.

31. Méthode selon l'une des revendications 23 à 30 où les anticorps utilisés dans la chromatographie par affinité d'anticorps sont élevés contre ou dirigés sensiblement uniquement contre des déterminants immunologiques des protéines d'interféron sous forme Le d'être humain selon l'une quelconque des revendications 1—9, et lesdits anticorps sont immobilisés sur une matrice.

32. Méthode selon la revendication 23 où les anticorps utilisés dans la charomatographie par affinité d'anticorps sont élevés contre ou dirigés sensiblement uniquement contre les déterminants immunologiques de protéines d'interféron sous forme Le d'être humain selon l'une quelconque des revendications 1—9, lesdits anticorps sont immobilisés sur une matrice et la solution appliquée à la matrice par affinité d'anticorps est une préparation brute en non concentrée d'interféron, ou bien une préparation concentrée ou partiellement purifiée.

33. Méthode selon la revendication 31 ou la revendication 32 où les anticorps sont d'abord obtenus en immunisant un animal immunisable avec des composants de protéines d'interféron sous forme Le d'être humain de 18.400±200, de 20.100±200 ou une combinaison de 18.400±200 et 20.100±200 Daltons, des protéines selon la revendication 1 ou la revendication 2.

34. Méthode selon l'une quelconque des revendications 31—33 où les anticorps sont liés de manière covalente à la matrice.

35. Méthode selon la revendication 34 où la matrice est un agarose réticulé tel que Sepharose 4B.

36. Méthode selon l'une quelconque des revendications 31—35 où les anticorps sont sensiblement libres d'activité enzymatique protéolytique.

37. Methode selon la revendication 36 où les anticorps ont été sensiblement libérés de toute activité enzymatique protéolytique par traitement avec des inhibiteurs ou destructeurs d'enzyme.

38. Méthode selon la revendication 37 où les anticorps ont été traités avec des inhibiteurs ou destructeurs d'enzyme immobilisés sur matrice.

39. Méthode selon la revendication 38 où les anticorps, avant leur liaison covalente à la matrice, sont passés à travers une colonne de poly-L-lysine immobilisée sur matrice et/ou d'inhibiteur de la Trypsine du soja immobilisé sur matrice et/ou d'inactivateur de kallikréine immobilisé sur matrice.

40. Méthode selon l'une quelconque des revendications 23—39 où l'interféron sous forme Le d'être humain de la solution soumise à une chromatographie par affinité d'anticorps est choisi dans le groupe consistant en interférons de leucocytes humains, interféron de lymphoblastoïdes humains et protéines selon la revendication 22.

41. Méthode de préparation des protéines d'interféron sous forme Le d'être humain selon la revendications 3—9 consistant à soumettre les protèines d'interféron sous forme Le d'être humain, selon la revendication 1 ou la revendication 2, aux conditions SDS PAGE définies ici, pour ainsi séparer les protéines selon leurs poids moléculaires et récupérer les composants individuels actifs de l'interféron de leurs positions relatives dans le gel PAGE.

Fig. 1.

EP 0 018 218 B1

20 300
20 100
18 400

1

Fig. 2.

Fig. 3

EP 0 018 218 B1

# Fig.4a.

45000

25000

17800

12400

1.0M NaCl
0.8M NaCl
0.6M NaCl
0.4M NaCl
0.2M NaCl
WASH
INPUT

# Fig.4.

EP 0 018 218 B1

Fig. 5.

Fig. 6.

7

# Fig. 7.

# Fig. 8.

# Fig. 9.

67 000

43 000

30 000

20 100

14 400

B      A

# Fig.10.